# EUROPEAN PATENT SPECIFICATION

(11) **EP 2 561 351 B1**
(45) Date of publication and mention of the grant of the patent: **09.12.2015**
(21) Application number: 11771678.7
(22) Date of filing: 22.04.2011
(51) Int. Cl.: G01N 33/48, C12Q 1/68, C40B 30/00, G01N 33/50, G01N 33/574, G01N 33/68

(54) **NOVEL BIOMARKERS AND TARGETS FOR OVARIAN CARCINOMA**
NEUE BIOMARKER UND ZIELE FÜR OVARIALKREBS
NOUVEAUX MARQUEURS BIOLOGIQUES ET CIBLES POUR LE CARCINOME OVARIEN

(30) Priority: 28.07.2010 US 368596 P; 22.04.2010 US 326859 P
(43) Date of publication of application: 27.02.2013
(73) Proprietor: British Columbia Cancer Agency Branch, Vancouver, British Columbia V5Z 1L3 (CA)
(72) Inventor: HUNTSMAN, David, G., Vancouver British Columbia V6N 3E9 (CA); MARRA, Marco, Vancouver British Columbia V5Z 1Y9 (CA); WIEGAND, Kimberly, Vancouver British Columbia V5N 4L7 (CA); HIRST, Martin, Delta British Columbia V4M 2R4 (CA); SHAH, Sohrab, Prakash, Vancouver British Columbia V5T 2M1 (CA)
(74) Representative: MacLean, Martin Robert
(86) International application number: PCT/IB2011/051763
(87) International publication number: WO 2011/132175

(56) References cited:
- WO-A2-2006/094068
- KYLIE L GORRINGE: "Mutation and Methylation Analysis of the Chromodomain- Helicase-DNA Binding 5 Gene in Ovarian Cancer1,2", NEOPLASIA, vol. 10, no. 11, November 2008 (2008-11), pages 1253-1258, XP055077659, DOI: 10.1593/neo.08718
- BOCHAR D A ET AL: "BRCA1 IS ASSOCIATED WITH A HUMAN SWI/SNF-RELATED COMPLEX: LINKING CHROMATIN REMODELING TO BREAST CANCER", CELL, CELL PRESS, US, vol. 102, 21 July 2000 (2000-07-21), pages 257-265, XP002949210, ISSN: 0092-8674, DOI: 10.1016/S0092-8674(00)00030-1
- WONG A K C ET AL: "BRG1, A COMPONENT OF THE SWI-SNF COMPLEX, IS MUTATED IN MULTIPLE HUMAN TUMOR CELL LINES", CANCER RESEARCH, AMERICAN ASSOCIATION FOR CANCER RESEARCH, US, vol. 60, no. 21, 1 November 2000 (2000-11-01), pages 6171-6177, XP001120675, ISSN: 0008-5472
- H. KOBAYASHI ET AL: "Risk of developing ovarian cancer among women with ovarian endometrioma: a cohort study in Shizuoka, Japan", INTERNATIONAL JOURNAL OF GYNECOLOGICAL CANCER, vol. 17, no. 1, 1 January 2007 (2007-01-01), pages 37-43, XP055077666, ISSN: 1048-891X, DOI: 10.1111/j.1525-1438.2006.00754.x
- HUANG ET AL.: 'Genomic and Functional Evidence for an ARIDIA Tumor Suppressor Role' GENES, CHROMOSOMES & CANCER vol. 46, 10 May 2007, pages 745 - 750, XP055098618
- LUO ET AL.: 'A genome-wide RNAi scree identifies multiple synthetic lethal interactions with the Ras oncogene' CELL vol. 137, no. 5, 29 May 2009, pages 835 - 848, XP055098615
- LUO ET AL.: 'Highly parallel identification of essential genes in cancer cells' PNAS vol. 105, no. 51, 23 December 2008, pages 20380 - 20385, XP055034447
- REISMAN ET AL.: 'The SWI/SNF complex and cancer' ONCOGENE vol. 28, 23 February 2009, pages 1653 - 1668, XP055098620
- WANG ET AL.: 'Expression of p270 (ARIDIA), a component of human SWI/SNF complexes,in human tumors' INT. J. CANCER vol. 112, 08 July 2004, pages 636 - 642, XP055098634
- WIEGAND ET AL.: 'ARIDIA mutations in Endometriosis-Associated Ovarian Carcinomas' N. ENGL. J. MED. vol. 363, no. 16, 14 October 2010, pages 1532 - 1543, XP055098627
- MAEDA ET AL.: 'Clinicopathological Significance of Loss of ARIDIA Immunoreactivity in Ovarian Clear Cell Carcinoma' INT.J.MOL.SCI. vol. 11, 13 December 2010, pages 5120 - 5128, XP055098626
- JONES ET AL.: 'Frequent Mutations of Chromatin Remodeling Gene ARIDIA in Ovarian Clear Cell Carcinoma' SCIENCE vol. 330, 08 October 2010, pages 228 - 231, XP055098629
- WIEGAND ET AL.: 'Loss of BAF250a (ARIDIA) is frequent in high-grade endometrial carcinomas' J. OF PATHOLOGY vol. 224, 18 May 2011, pages 328 - 333, XP055098632

## Description

### Reference to Related Applications

This application claims the benefit of U.S. provisional patent application No. 61/326,859 filed 22 April 2010 and U.S. provisional patent application No. 61/368,596, filed 28 July 2010, both entitled NOVEL MARKERS AND THERAPEUTIC TARGETS FOR CLEAR CELL CARCINOMA OF THE OVARY.

### Technical Field

Embodiments of this invention relate to diagnosis, prognosis, and predicting response to treatment of certain types of cancer. The disclosure also relates to improved methods for therapy, and to methods for screening for and developing novel targets, biomarkers and therapeutics for treating certain types of cancer. Embodiments of the invention have particular application in diagnosis, prognosis and predicting response to treatment of clear cell carcinoma of the ovary, endometrioid carcinoma, endometrial carcinoma and uterine carcinoma. Also disclosed are methods for therapy, methods of screening for and developing novel therapeutics for treating clear cell carcinoma of the ovary, endometrioid carcinoma, and uterine carcinoma.

### Background

In North America, ovarian cancer is the leading cause of death due to gynaecological malignancies and is the fifth leading cause of cancer death in Canadian women. Ovarian cancers can be divided into subtypes based on their tumour cell types. Clear cell carcinomas (CCC) of the ovary are one of the ovarian cancer subtypes and represent approximately 12% of all malignant ovarian tumours. Though they are intrinsically resistant to traditional platinum and taxane therapies, these cancers are still treated similarly to other ovarian cancers. Patients with CCC are therefore exposed to treatment which is ineffective, toxic, and expensive and there are currently no alternative anti-cancer agents effective for this disease. Thus, due to the limited success of traditional chemotherapy, there is an urgent need for more effective treatments which are specific to the CCC subtype of ovarian cancer.

### Epithelial Ovarian Cancer

Epithelial ovarian cancer is the fifth leading cause of cancer death and second most common gynaecological malignancy in Canada. There are several subtypes of epithelial ovarian cancer. High grade serous cancers are the most common and account for approximately 70% of all cases. CCCs are the second most common subtype (12% of cases)² and the second leading cause of ovarian cancer associated deaths. Whereas high grade serous cancers are the subject of The Cancer Genome Atlas Project, CCCs are relatively understudied.

### Clinical, Pathological, and Molecular Characteristics of Clear Cell Carcinomas

Despite evidence that ovarian carcinoma subtypes are essentially different diseases^{3, 4}, it is current practice to treat them all with platinum/taxane chemotherapy. CCCs, however, respond extremely poorly to this treatment⁵⁻⁷ with response rates of 15% compared to 80 % for high grade serous carcinomas⁴. CCCs have a low mitotic rate^{4,8}, are genetically stable, diploid or tetraploid and develop from well-established precursor lesions. They do not exhibit the complex karyotypes or chromosomal instability associated with high grade serous cancers^{8,9}, which may contribute to their chemoresistance. CCCs are often diagnosed at an early stage, with 80% of cases presenting with stage I or II carcinoma^{10,11}, however survival rates for stage I/II CCC are significantly lower (60%) compared to patients with other ovarian cancer subtypes presenting with stage **I/II** disease^{7,12}. There are currently no effective anti-cancer agents for CCCs.

CCCs are defined based on histopathological findings as tumours composed predominantly of clear cells and hobnail cells¹³. While CCC express hepatocyte nuclear factor-1beta, they rarely express biomarkers commonly associated with high grade serous or other ovarian cancers⁴ and the distinctive CCC immunophenotype can be used as an aid in diagnostically challenging cases¹⁴. The most commonly mutated gene in CCC is *PIK3CA* (present in 14%-50% of cases)¹⁵⁻¹⁹. By contrast, *BRCA1*, *BRCA2,* and *TP53* mutations are commonly found in high grade serous cancers but are typically absent in CCCs^{19,20}. Though there is an association between both CCCs and low-grade endometrioid carcinomas with endometriosis²¹, the mechanism of this transformation was previously unknown for CCCs. In addition, CCCs can arise from adenofibromas^{22,23}. CCCs are aggressive cancers untreatable with current chemotherapy, are poorly understood, and remain relatively understudied. In addition, they are genomically stable^{8,9}.

### Next generation sequencing

Next generation sequencing technology is based on massively parallel single molecule sequencing to cost-effectively produce millions of short sequence reads. This technology can fully interrogate genomes or transcriptomes at a single base resolution for single nucleotide variance, splice variants, genome rearrangements, copy number changes, inversions, and insertions and deletions²⁴. In the case of paired-end sequencing, next generation sequencing technology generates millions of randomly fragmented, short sequenced reads that flank longer unsequenced regions. Data is generated using a four-color DNA "sequencing-by-synthesis" technology followed by fluorescence detection. After completion of the first read, templates are regenerated *in situ* to enable a second read from the opposite end of the fragments, producing end-sequence pairs. It is possible to use this technology for whole genome analysis, however this is much more costly than RNA-seq (whole transcriptome analysis) which sequences cDNAs generated from total mRNA. Resulting paired-end reads are aligned to a reference sequence (e.g. NCBI build 36.1, hg18) which produces relevant data on each read, such as location within the transcriptome, quality of read, number of mismatches, and paired-end flags. Single nucleotide variants (SNVs) are predicted based on discrepancies between the reference genome and the aligned mapped reads. Fusion transcripts and other rearrangements are recognized by identifying all mate-pairs that do not align canonically in pairs to the human genome.

### The SWI/SNF Complex

Chromosomal DNA is wound around proteins called histones to form a complex structure called chromatin. The basic unit of chromatin is the nucleosome which is composed of DNA wrapped around eight histone proteins. Nucleosomes are connected by linker DNA, similar to beads on a string. Further coiling or condensation of chromatin creates a higher order structure known as heterochromatin. DNA organized into heterochromatin is inaccessible to transcriptional machinery. Chromatin remodelling, either through covalent modification of histones or through the mobilization of nucleosomes, is required before DNA can be accessed for transcriptional initiation.

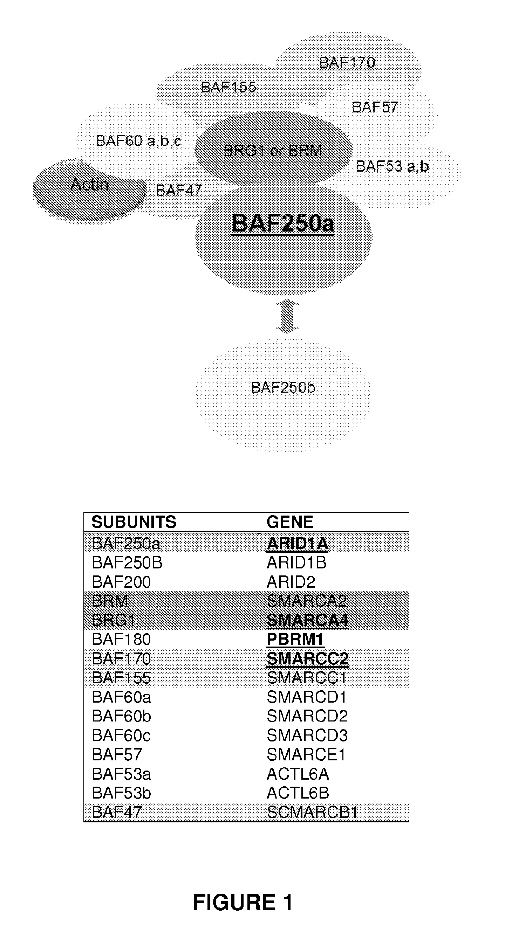

The SWI/SNF protein complex uses ATP hydrolysis to mobilize nucleosomes which modulates accessibility to transcription machinery. The SWI/SNF protein complex is typically associated with transcriptional activation or repression and functions at the promoter. This complex is present in all eukaryotes and is essential for many cellular processes including development, differentiation, proliferation, DNA repair, and tumour suppression²⁶. The complex is comprised of one of two ATPases, BRM (Brahma) or BRG1 (Brahma-Related Gene 1)^{27,28}, along with conserved core subunits and variable accessory proteins termed BAFs (BRM- or BRG1- associated factors) (Figure 1). The specific combination of proteins within different complexes is believed to confer specificity with respect to gene regulation.

BRG1 containing SWI/SNF complexes contain either BAF250 or BAF180, while BRM complexes contain only BAF250. There are two BAF250 proteins which are encoded by paralogous genes. BAF250a (also referred to as p270) is encoded by the *ARID1A* gene and BAF250b is encoded by the *ARID1B* gene. These proteins are mutually exclusive within BRG1 or BRM containing SWI/SNF complexes²⁹. Co-immunoprecipitation studies indicate that BAF250a and BAF250b interact with BRG1 and BRM through their C-terminal domains³⁰ and the interaction between BAF250a and BRG1 has been shown to be required for transactivation of the MMTV (mouse mammary tumour virus) promoter³¹. This steroid hormone responsive promoter is often used as part of a model system to study transcriptional activation from SWI/SNF-mediated chromatin remodelling. Specifically, BAF250a has been shown to stimulate glucocorticoid receptor-mediated transactivation; this requires the presence of the BAF250a C-terminus which can directly interact with the glucocorticoid receptor in vitro³². Huang et al. (Genes, Chromosomes & Cancer, 46:745-750) suggests that *IRID1A* is a tumor suppressor gene.

There remains an unmet need in the oncology field for new treatment modalities that specifically target the molecular defects driving the pathogenensis of CCC, endometrioid carcinoma (EC), and uterine carcinoma. There is a need for novel prognostic, diagnostic and predictive (response to treatment) markers for CCC, EC, and uterine carcinoma. There is a need for novel therapeutic targets for treatment of CCC, EC, and uterine carcinoma, methods for identifying such novel therapeutic targets, and therapeutic agents for treating these cancers.

### Summary

The following embodiments and aspects thereof are described and illustrated in conjunction with systems, tools and methods which are meant to be exemplary and illustrative, not limiting in scope. In various embodiments, one or more of the above-described problems have been reduced or eliminated, while other embodiments are directed to other improvements.

The invention is defined according to the claims.

In one aspect, the invention provides a method for determining whether endometriosis of a subject is likely to progress to clear cell ovarian carcinoma, endometrioid carcinoma, endometrial carcinoma, or uterine carcinoma; for determining the prognosis for a subject suffering from clear cell ovarian carcinoma, endometrioid carcinoma, endometrial carcinoma, or uterine carcinoma; and/or for determining whether standard chemotherapeutic agents are likely to be effective in treating clear cell ovarian carcinoma, endometrioid carcinoma, endometrial carcinoma, or uterine carcinoma; the method comprising assaying a sample of the endometriosis or carcinoma for expression of BAF250a, wherein the absence of expression of BAF250a indicates a likelihood that the endometriosis will progress to clear cell ovarian carcinoma, endometrioid carcinoma, endometrial carcinoma, or uterine carcinoma, indicates a poor prognosis, or indicates that the standard chemotherapeutic agents are not likely to be effective.

In another aspect, the invention provides a method for determining whether endometriosis of a subject is likely to progress to clear cell ovarian carcinoma, endometrioid carcinoma, endometrial carcinoma, or uterine carcinoma; for determining a prognosis for a subject suffering from clear cell ovarian carcinoma, endometrioid carcinoma, endometrial carcinoma, or uterine carcinoma; and/or for determining whether standard chemotherapeutic agents are likely to be effective in treating clear cell ovarian carcinoma, endometrioid carcinoma, endometrial carcinoma, or uterine carcinoma; the method comprising assaying a sample of the endometriosis or carcinoma for the presence of mutations in the ARID1A gene in the sample, wherein the presence of a significant mutation in the ARID1A gene indicates a likelihood that the endometriosis will progress to clear cell ovarian carcinoma, endometrioid carcinoma, endometrial carcinoma, or uterine carcinoma, indicates a poor prognosis, or indicates that the standard chemotherapeutic agents are not likely to be effective, wherein the significant mutation comprises a mutation in the DNA sequence of the ARID1A gene that produces a mutated BAF250a protein that is not able to fully perform the typical function of BAF250a.

Embodiments of the invention provide novel biomarkers and therapeutic targets for treatment of certain types of cancer, including CCC, EC, endometrial carcinoma, and uterine carcinoma. Mutations in genes encoding proteins that form part of the SWI/SNF chromatin remodelling protein complex, including ARID1A, or loss of expression of such proteins, including BAF250a, can be used to evaluate the likelihood endometriosis will progress or transform to cancer, to provide a prognosis for a patient with cancer, to assess whether conventional treatment is likely to be effective against a cancer, and/or in a synthetic lethal screen to identify novel targets and therapeutics for the treatment of cancer.

Mutations in *ARID1A* or other genes encoding proteins that are components of the SWI/SNF complex can be assessed by assaying for the presence of such mutations in a sample of tissue obtained from a site of endometriosis or a carcinoma of a subject. Techniques that may be used to confirm the presence of mutations in *ARID1A* include Sanger sequencing of the tissue sample or next generation sequencing of the tissue sample, PCR-based methods including Amplification Refractory Mutation System (ARMS)-based PCR, or TaqMan^{™} assays, or hybridization-based methods including fluorescence *in-situ* hybridization (FISH), or any other suitable detection technique.

Loss of expression of proteins that are components of the SWI/SNF complex, including BAF250a, can be assessed by obtaining a sample of tissue from a site of endometriosis or a carcinoma of a subject for expression of that protein, for example using immunohistochemistry.

In some aspects of the disclosure, cells having mutations in *ARID1A* or other genes encoding proteins that are components of the SWI/SNF complex can be used in a synthetic lethal screen to identify new targets for the treatment of CCC, EC and uterine carcinoma. Targets identified by such screens can be used to screen for novel therapeutics useful in the treatment of CCC, EC and uterine carcinoma.

In addition to the exemplary aspects and embodiments described above, further aspects and embodiments will become apparent by reference to the drawings and by study of the following detailed descriptions.

### Brief Description of Drawings

Exemplary embodiments are illustrated in referenced figures of the drawings. It is intended that the embodiments and figures disclosed herein are to be considered illustrative rather than restrictive.
Figure 1 shows a schematic overview of the protein components of the SWI/SNF complex and lists the fifteen genes that encode components of the SWI/SNF complex.

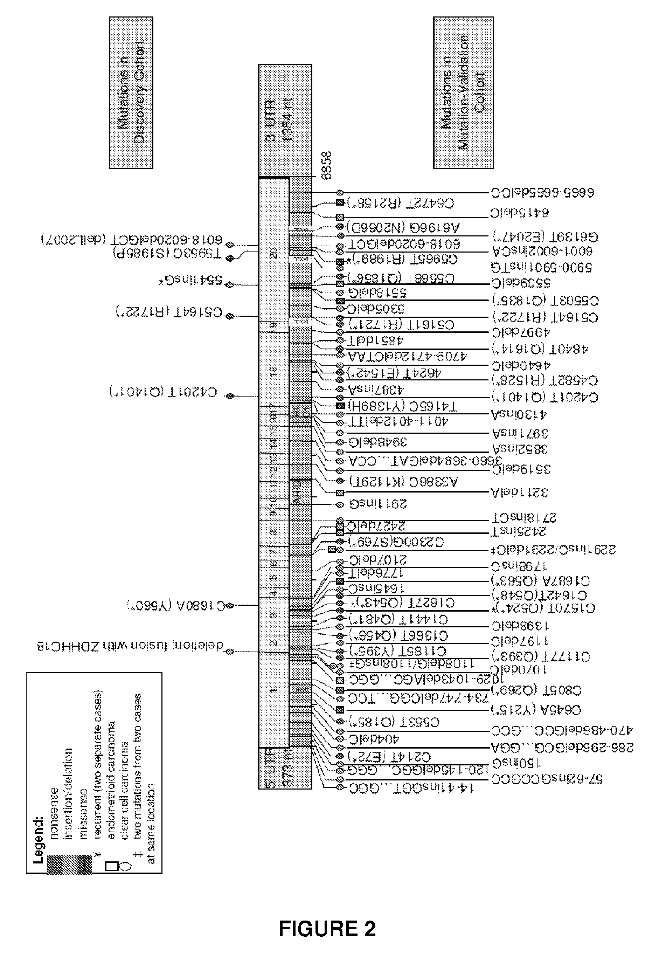

Figure 2 shows a schematic overview of the ARID1A cDNA (from ATG start to TGA stop) and BAF250a protein. Mutations identified by the inventors by transcriptome (RNA) sequencing are summarized above the schematic. Mutations identified by the inventors by targeted exon resequencing and Sanger sequencing of genomic DNA are shown below the schematic. Numbers 1 through 6858 below the schematic indicate the nucleotide (nt) position, starting with the A in the ATG start codon for ARID1A in position 1 (based on the sequence given in record number NM_006015.4 in Entrez Gene). UTR denotes untranslated region.
Figure 3 summarizes mutations identified by RNA sequencing and exon resequencing of 19 specimens of CCC.
Figure 4 summarizes the results of sequence analysis, tumor and germline validation, and BAF250a expression measured for samples exhibiting mutations in *ARID1A.* The SEQ ID NO. of each mutant gene sequence is listed.
Figure 5 summarizes mutations in genes other than *ARID1A* identified by RNA sequencing of 19 specimens of CCC.
Figure 6 shows BAF250a expression, *ARID1A* mutations, and loss of heterozygosity in CCC23 and corresponding endometriotic precursor lesions.
Figure 7 shows results of Sanger sequencing, RNA sequencing, and immunohistochemical staining of BAF250a in case CCC 14.
Figure 8 shows immunofluorescence demonstrating knockdown of BAF250a expression through stable expression *ARID1A* shRNA in HCT116 cells. Picture taken at 63X magnification.
Figure 9 shows the correlation between *ARID1A* mutation status and the presence of endometriosis at the time of surgery for 119 samples of CCC and EC.
Figure 10 lists the primer sequences used for validating the sequence of *ARIDIA* by targeted exon resequencing.
Figure 11 shows the sequence prediction for the ARID1A-ZDHHC18 fusion identified by RNA sequencing.
Figure 12 shows the mutational status and corresponding expression of BAF250a in the discovery and mutation-validation cohorts according to carcinoma type.
Figure 13 shows BAF250a expression in tumors (with the number and total number in parentheses) from three subtypes of ovarian cancer -- clear-cell carcinoma (CCC), endometrioid carcinoma (EC), and high-grade serous (HGS) carcinoma.
Figure 14 shows experimental results for CCC23 and adjacent atypical endometriosis.
Figure 15 shows the results of analysis of clear cell carcinoma from specimen CCC13 and adjacent atypical endometriosis.
Figure 16 shows Sanger sequencing results from CCC13.
Figure 17 shows a table summarizing the results of immunohistochemstry for BAF250a expression in the tissue microarrays studied.

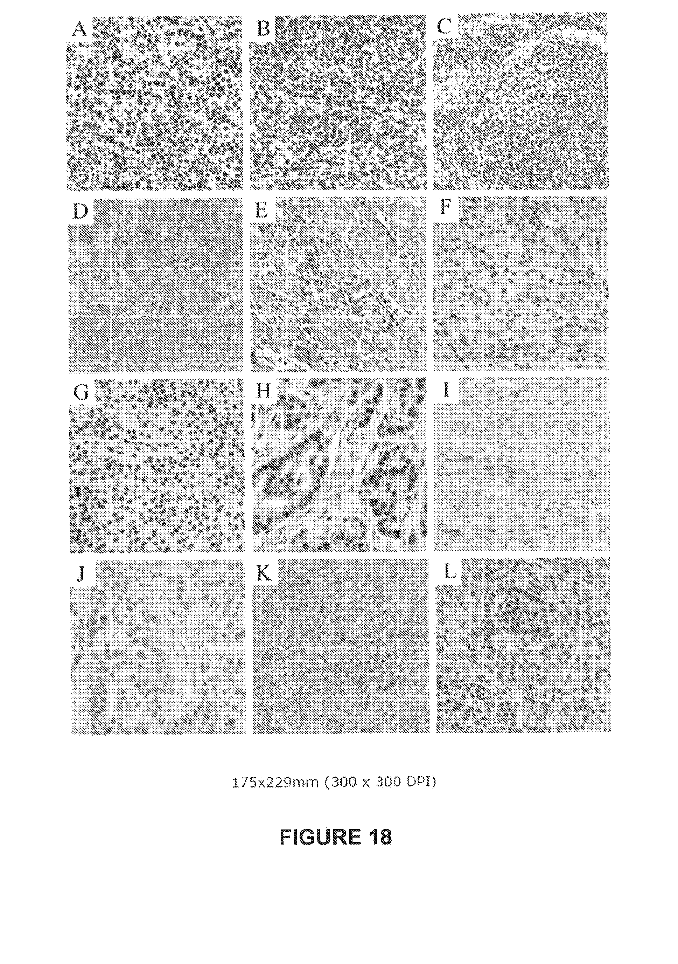

Figure 18 shows immunostaining for BAF250a expression in diverse malignancies, including: (A) DLBCL (diffuse large B-cell lymphoma), (B) MCL (mantle cell lymphoma), (C) follicular lymphoma, (D) oral cancer, (E) gastric cancer, (F) anaplastic thyroid cancer, (G) renal cancer, (H) pancreatic cancer, (I) GIST (gastrointestinal stromal tumor), (J) breast cancer, (K) cervical cancer, and (L) sex cord-stromal tumours. Loss of BAF250a is demonstrated in gastric cancer (E), as shown by lack of tumour cell staining and positive stromal staining; all other panels demonstrate positive BAF250a staining. Images were captured at 20X magnification.
Figure 19 shows that high-grade malignancies of the endometrium show loss of BAF250a expression. Tissue cores of (A) high-grade endometroid carcinoma, (B) clear cell carcinoma, (C) high-grade serous carcinoma, and (D) carcinosarcoma. For all panels, note the lack of BAF250a immunostaining in the tumour cells, while the adjacent nonneoplastic stromal cells show positive BAF250a nuclear staining. Original magnification for all panels, 20X.

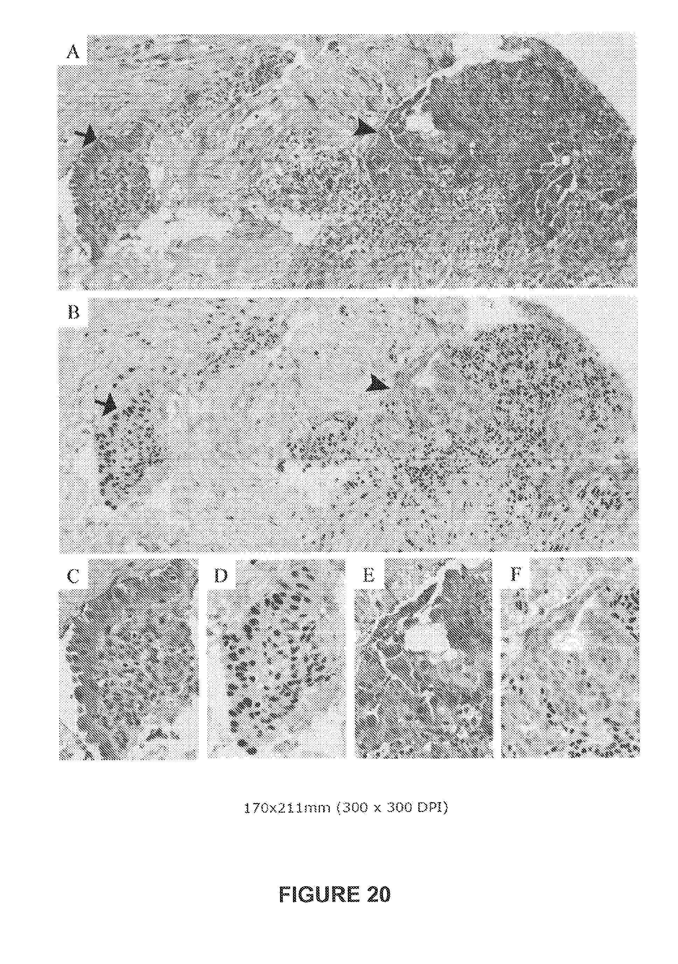

Figure 20 shows a biopsy from cul-de-sac showing endometriosis, with endometrial-type glands and stroma. (A) On H&E staining there is focal cytological atypia of the glandular epithelium (arrowhead), while other glandular epithelial cells do not show atypia (arrow). (B) Immunostaining for BAF250a shows loss of expression in the glandular epithelial cells of atypical endometriosis (arrowhead), with expression in non-atypical glandular epithelial cells and in endometrial stromal cells (arrow). Panels (A) and (B) were captured at 20X. Panels (C) and (D) show 40X magnification for the H&E and BAF250a IHC, respectively, for the nonatypical glandular epithelial cells. Panels (E) and (F) show 40X magnification for the H&E and BAF250a IHC, respectively, for the atypical endometriosis.
Figure 21 shows the 50 genes found to have the greatest differential expression versus wild type in cells having an *ARID1A* mutation.
Figure 22 shows a flowchart for experiments that will be conducted to assess the effect of *ARID1A* mutations on cell growth.

### Description

Throughout the following description specific details are set forth in order to provide a more thorough understanding to persons skilled in the art. However, well known elements may not have been shown or described in detail to avoid unnecessarily obscuring the disclosure. Accordingly, the description and drawings are to be regarded in an illustrative, rather than a restrictive, sense.

For further clarity, database identifiers for the *ARID1A* gene, RNA and protein are as follows: Entrez Gene: 8289; UniProtKB/Swiss-Prot: ARI1A_HUMAN, 014497; RefSeq DNA sequence: NC_000001.10 NT_004610.19; REFSEQ mRNAs for *ARIDIA* gene (2 alternative transcripts): NM_006015.4 NM_139135.2. The wild-type sequence for *ARID1A* (NM_006015.4) is set forth in SEQ ID NO.:1.

The inventors have now discovered that mutations in genes encoding proteins that are components of the SWI/SNF complex are useful as biomarkers or targets to assist in the diagnosis, prognosis and treatment of, and development of therapeutic agents for, certain types of cancer including clear cell carcinoma (CCC) of the ovary, endometrioid carcinoma (EC), and uterine carcinoma. The inventors have demonstrated that such mutations are relatively common in endometrial carcinomas but relatively infrequent in other types of cancer. The mechanism of progression of cancer involving these mutations appears to be distinct from other known mechanisms of cancer development. See also Wiegand et al., N. Engl. J. Med. 2010, 363:1532-1543, and the Supplementary Appendix thereto.

Ovarian CCC and EC are thought to arise from endometriosis. The presence of nonsense mutations, significant missense mutations, or genetic rearrangements in genes encoding proteins that are important to the proper functioning of the SWI/SNF complex in endometriosis may indicate a risk of malignant progression or transformation of endometriosis to these cancers or other types of ovarian cancers, a poor prognosis for a patient having a form of cancer with such mutations, or a likelihood that standard chemotherapeutic agents such as platinum or taxane therapeutics are unlikely to be effective in treating a form of cancer with such mutations. A lack of expression of proteins that are important to the proper functioning of the SWI/SNF complex in endometriosis may indicate a risk of malignant progression or transformation of endometriosis to these cancers or other types of ovarian cancers. A lack of expression of proteins that are important to the proper functioning of the SWI/SNF complex in a carcinoma may indicate a poor prognosis for a patient with the carcinoma, and/or a likelihood that standard chemotherapeutic agents such as platinum or taxane therapeutics are unlikely to be effective in treating that carcinoma.

As used herein, the term "significant mutation" when used with reference to a gene means a mutation in the DNA sequence of the gene that produces a mutated protein that is not able to fully perform the typical function of that protein. The term "significant mutation" when used with reference to a protein means a mutation in the DNA sequence encoding that protein that produces a mutated protein product that is not able to fully perform the typical function of that protein, and includes all mutations equivalent thereto by reason of the degeneracy of the genetic code. A significant mutation could include a truncation mutation, a nonsense mutation, a significant missense mutation, and/or a genetic rearrangement.

As used herein, the term "poor prognosis" means a significant prospect that a patient with cancer will suffer a negative outcome, e.g. morbidity or death, as a result of the cancer.

Embodiments of the invention provide novel targets and molecular defects associated with the development and pathogenesis of CCC of the ovary, EC, endometrial carcinoma and uterine carcinoma. These targets and defects are distinct from those characteristic of other types of ovarian cancer and will enable the development of new therapies effective for treatment of CCC of the ovary, EC and uterine carcinoma.

Embodiments of the invention provide novel biomarkers useful for the prognosis of CCC of the ovary, EC, endometrial carcinoma and uterine carcinoma. Embodiments of the invention provide novel biomarkers to enable prediction of the risk of malignant progression (or transformation) of endometriotic lesions (endometriosis) to these cancers or other types of ovarian cancer.

Embodiments of the invention provide novel biomarkers useful for predicting response to treatment (chemotherapy, radiation, targeted drug therapy and the like) of patients with CCC of the ovary, EC, endometrial carcinoma and uterine carcinoma.

Mutations in one or more of the other genes/proteins comprising the SWI/SNF chromatin remodelling complex are markers that are useful as therapeutic targets, or to enable the development of therapeutic targets for treatment of CCC of the ovary, EC and uterine carcinoma.

Mutations in one or more of the other genes/proteins comprising the SWI/SNF chromatin remodelling complex are novel biomarkers useful for the prognosis of CCC of the ovary, EC and uterine carcinoma and for prediction of the risk of malignant progression (or transformation) of endometriotic lesions (endometriosis).

In another aspect of the disclosure, mutations in one or more of the genes/proteins comprising the SWI/SNF chromatin remodelling complex are novel biomarkers that are useful for predicting response to treatment (chemotherapy, radiation, targeted drug therapy and the like) of patients with CCC of the ovary, EC and uterine carcinoma.

In another aspect of the disclosure, one or more mutations in the gene *ARID1A* (encoding protein BAF250a (also referred to as p270)), a component of the SWI/SNF chromatin remodelling complex, are markers that are useful as therapeutic targets, or to enable the development of therapeutic targets for treatment of CCC of the ovary, EC and uterine carcinoma.

In another aspect of the invention, one or more mutations in the gene *ARID1A* (encoding protein BAF250a (also referred to as p270)), a component of the SWI/SNF chromatin remodelling complex, are novel biomarkers useful for the prognosis of CCC of the ovary, EC, endometrial carcinoma and uterine carcinoma and for prediction of the risk of malignant progression (or transformation) of endometriotic lesions (endometriosis).

In another aspect of the invention, one or more mutations in the gene *ARID1A* (encoding protein BAF250a (also referred to as p270)), a component of the SWI/SNF chromatin remodelling complex, are novel biomarkers that are useful for predicting response to treatment (chemotherapy, radiation, targeted drug therapy and the like) of patients with CCC of the ovary, EC, endometrial carcinoma and uterine carcinoma.

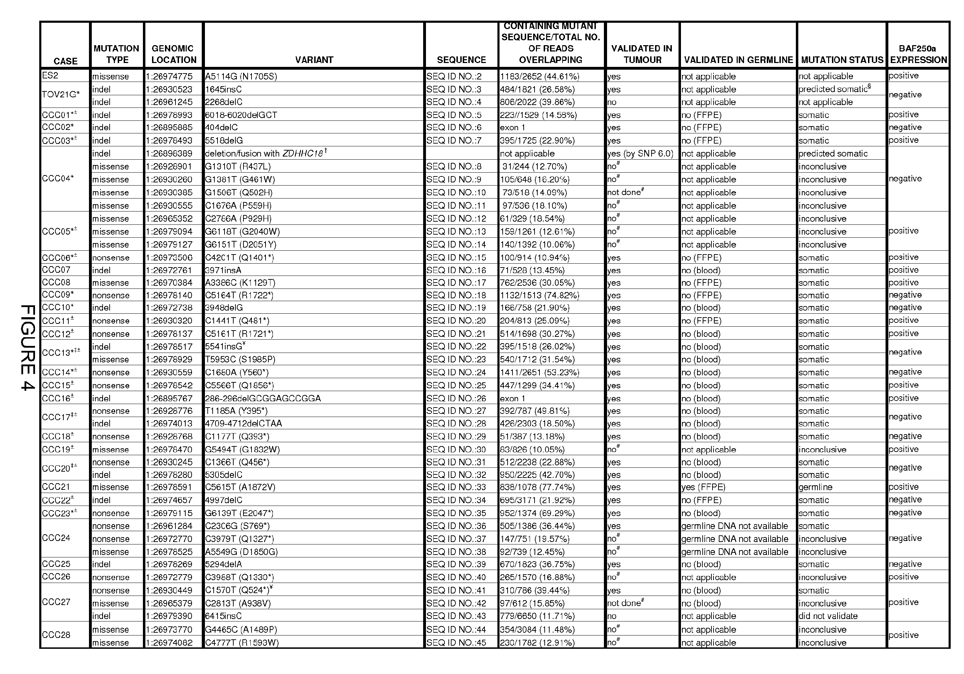

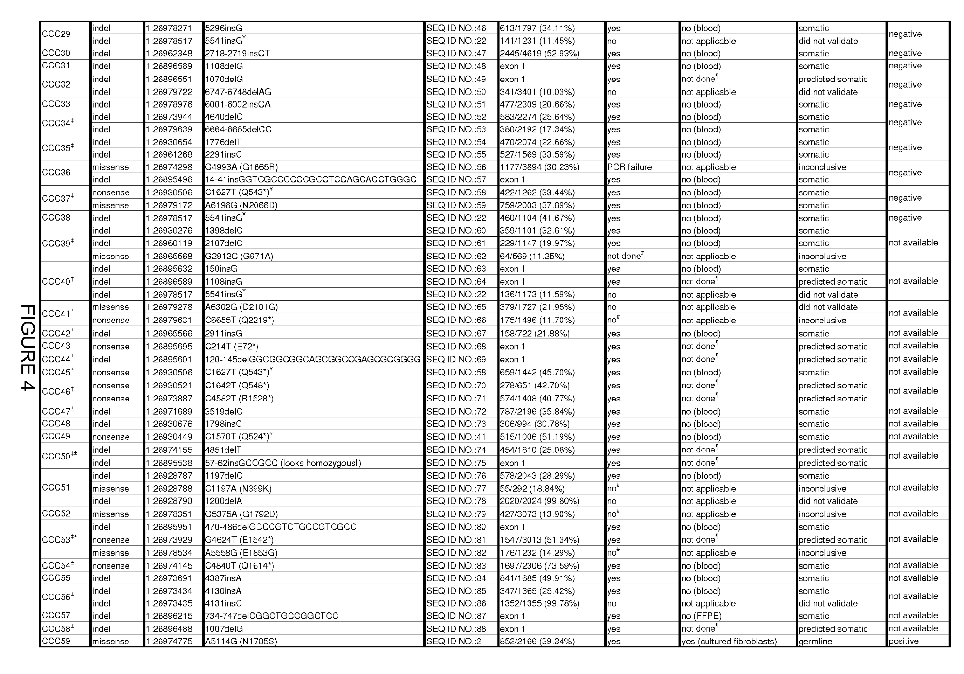

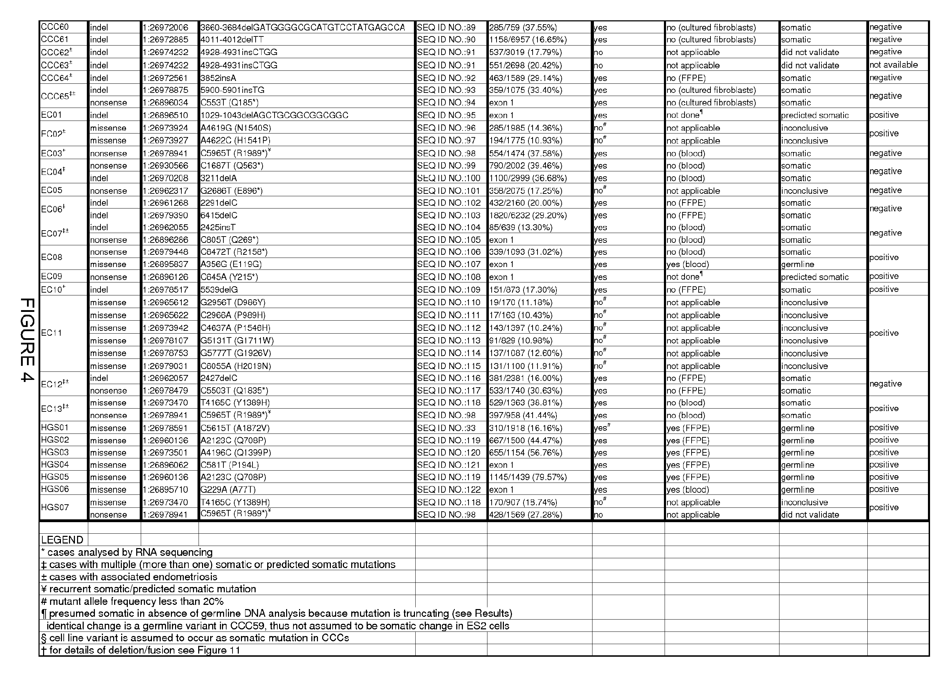

In an aspect of the disclosure, one or more of the mutations in SEQ ID NO.:2 through SEQ ID NO.: 122 (shown in Figure 4 of this specification) in the gene *ARID1A* (encoding protein BAF250a (also referred to as p270)), a component of the SWI/SNF chromatin remodelling complex, are markers that are useful as therapeutic targets, or to enable the development of therapeutic targets for treatment of CCC of the ovary, EC and uterine carcinoma.

In another aspect of the invention, one or more of the mutations in SEQ ID NO.:2 through SEQ ID NO.: 122 (shown in Figure 4 of this specification) in the gene *ARID1A* (encoding protein BAF250a (also referred to as p270)), a component of the SWI/SNF chromatin remodelling complex, are novel biomarkers useful for the prognosis of CCC of the ovary, EC, endometrial carcinoma and uterine carcinoma and for prediction of the risk of malignant progression (or transformation) of endometriotic lesions (endometriosis).

In an aspect of the invention, one or more of the mutations in SEQ ID NO.:2 through SEQ ID NO.: 122 (shown in Figure 4 of this specification) in the gene *ARID1A* (encoding protein BAF250a (also referred to as p270)), a component of the SWI/SNF chromatin remodelling complex, are novel biomarkers that are useful for predicting response to treatment (chemotherapy, radiation, targeted drug therapy and the like) of patients with CCC of the ovary, EC, endometrial carcinoma and uterine carcinoma.

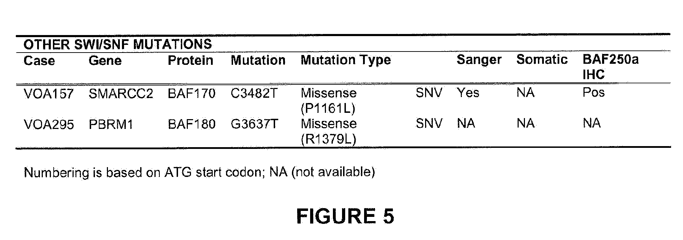

In an aspect of the invention, one or more mutations (shown in Figure 5 of this specification) in the genes *SMARCA4* (encodes for the protein BRG1), *PBRM1* (encodes for the protein BAF180) or *SMARCC2* (encodes for the protein BAF170), all components of the SWI/SNF chromatin remodelling complex, are markers that are useful as therapeutic targets, or to enable the development of therapeutic targets for treatment of CCC of the ovary, EC and uterine carcinoma.

In another aspect of the invention, one or more mutations (shown in Figure 5 of this specification) in the genes *SMARCA4* (encodes for the protein BRG1), *PBRMI* (encodes for the protein BAFI80) or *SMARCC2* (encodes for the protein BAF170), all components of the SWI/SNF chromatin remodelling complex, are novel biomarkers useful for the prognosis of CCC of the ovary, EC and uterine carcinoma and for prediction of the risk of malignant progression (or transformation) of endometriotic lesions (endometriosis).

In another aspect of the invention, one or more mutations (shown in Figure 5 of this specification) in the genes *SMARCA4* (encodes for the protein BRG1), *PBRMI* (encodes for the protein BAF180) or *SMARCC2* (encodes for the protein BAF170), all components of the SWI/SNF chromatin remodelling complex, are novel biomarkers that are useful for predicting response to treatment (chemotherapy, radiation, targeted drug therapy and the like) of patients with CCC of the ovary, EC and uterine carcinoma.

The presence of mutations in one or more genes that encode components of the SWI/SNF complex that disrupt the function or expression of the corresponding protein products in a sample of tissue obtained from a pre-cancerous lesion of a subject indicates a risk of malignant progression or transformation of the lesion to cancer. The presence of mutations in such genes can be determined by any suitable method, such as, for example, Sanger sequencing of the tissue sample or next generation sequencing of the tissue sample, PCR-based methods including ARMS-based PCR, fluorescence *in situ* hybridization (FISH), or other suitable detection technique. In some embodiments of the invention, the one or more gene includes *ARID1A*. In some aspects of the disclosure, the one or more genes are *ARID1B, ARID2, SMARCA2, SMARCC1, SMARCD1, SMARCD2, SMARCD3, SMARCE1, ACTL6A, ACTL6B, or SCMARCB1*.

The absence of expression of one or more proteins that are components of the SWI/SNF complex in a sample of tissue obtained from a pre-cancerous lesion of a subject indicates a risk of malignant progression or transformation of the pre-cancerous lesion to cancer. The expression level of the proteins in the tissue sample may be determined in any suitable manner, including for example immunohistochemistry. In some embodiments of the invention, the one or more proteins includes BAF250a. In some aspects of the disclosure, the proteins are BAF250b, BAF200, BRM, BAFI55, BAF60a, BAF60b, BAF60c, BAF57,BAF53a, BAF53b, or BAF47.

In some embodiments, the presence of mutations in *ARID1A,* a gene encoding the protein BAF250a, that disrupt the function or expression of BAF250a in a sample of tissue obtained from an endometriotic lesion of a subject indicates a risk of malignant progression or transformation of the endometriotic lesion to cancers such as CCC, EC, endometrial carcinoma or uterine cancer. The presence of mutations in *ARID1A* in the tissue sample can be determined by any suitable method, such as, for example, Sanger sequencing of the tissue sample or next generation sequencing of the tissue sample, PCR-based methods including ARMS-based PCR, FISH, or other suitable detection technique.

In some embodiments, the mutations in *ARID1A* that indicate a risk of malignant progression or transformation of the endometriotic lesion to cancers such as CCC, EC, endometrial carcinoma or uterine cancer include the mutations set forth in SEQ ID NO.: 2 through SEQ ID NO.: 122 (shown in Figure 4).

In some embodiments, the absence of expression of BAF250a in a sample of tissue obtained from an endometriotic lesion of a subject indicates a risk of malignant progression or transformation of the endometriotic lesion to cancers such as CCC, EC, endometrial carcinoma or uterine cancer. The expression level of BAF250a in the tissue sample may be determined in any suitable manner, including for example immunohistochemistry.

In some embodiments, the mutations in BAF250a that indicate a risk of malignant progression or transformation of the endometriotic lesion to cancers such as CCC, EC, endometrial carcinoma or uterine cancer include the mutations set forth in Figure 4.

The presence of mutations in *SMARCA4, PBRM1,* or *SMARCC2* that disrupts the function or expression of BRG1, BAF180, or BAF170, respectively, in a sample of tissue obtained from an endometriotic lesion of a subject indicates a risk of malignant progression or transformation of the endometriotic lesion to cancers such as CCC, EC or uterine cancer. The presence of mutations in these genes in the tissue sample can be determined by any suitable method, such as, for example, Sanger sequencing of the tissue sample or next generation sequencing of the tissue sample, PCR-based methods including ARMS-based PCR, FISH, or other suitable detection technique.

The absence of expression of BRG1, BAF180, or BAF170 in a sample malignant progression or transformation of the endometriotic lesion to cancers such as CCC, EC or uterine cancer. The expression level of BRG1, BAF180 or BAF170 in the tissue sample may be determined in any suitable manner, including for example immunohistochemistry.

The presence of mutations in one or more genes that encode components of the SWI/SNF complex that disrupt the function or expression of the corresponding protein products in a sample of tissue obtained from a cancerous lesion of a subject indicates a poor prognosis for the subject. The presence of mutations in such genes can be determined by any suitable method, such as, for example, Sanger sequencing of the tissue sample or next generation sequencing of the tissue sample, PCR-based methods including ARMS-based PCR, or TaqMan^{™} assays, or hybridization-based methods including FISH, or any other suitable detection technique. In some aspects of the disclosure, the one or more genes are *ARID1B, ARID2, SMARCA2, SMARCC1, SMARCD1, SMARCD2, SMARCD3, SMARCE1*, *ACTL6A, ACTL6B,* or *SCMARCB1*.

. The absence of expression of one or more proteins that are components of the SWI/SNF complex in a sample of tissue obtained from a cancerous lesion of a subject indicates a poor prognosis for the subject. The expression level of the proteins in the tissue sample may be determined in any suitable manner, including for example immunohistochemistry. In some embodiments of the invention, the one or more proteins includes BAF250a. In some aspects of the disclosure, the proteins are BAF250b, BAF200, BRM, BAF155, BAF60a, BAF60b, BAF60c, BAF57, BAF53a, BAF53b, or BAF47.

In some embodiments, the presence of mutations in *ARID1A*, a gene encoding the protein BAF250a, that disrupt the function or expression of BAF250a in a sample of tissue obtained from a CCC, EC, endometrial carcinoma, or uterine cancer of a subject indicates a poor prognosis for the subject. The presence of mutations in *ARID1A* in the tissue sample can be determined by any suitable method, such as, Sanger sequencing of the tissue sample or next generation sequencing of the tissue sample, PCR- based methods including ARMS-based PCR, or TaqMan™ assays, or hybridization-based methods including FISH, or any other suitable detection technique.

Those skilled in the art will recognize that a number of methods or techniques for identifying products such as ARMS-PCR products may be used in order to detect the presence of mutations in *ARID1A* or other genes encoding proteins that are components of the SWI/SNF complex. For example, embodiments include, but are not limited to, techniques such as primer extension, classical microarrays or line probes. Methods of PCR product endpoint detection including, but not limited to, fluorescence, chemiluminescence, colourimetric techniques or measurement of redox potential may also be used with the embodiments described herein for detecting gene mutations.

In some embodiments, the mutations in *ARID1A* that indicate a poor prognosis include the mutations in SEQ ID NO.:2 through SEQ ID NO.: 122, set forth in Figure 4.

In some embodiments, the absence of expression of BAF250a in a sample of tissue obtained from a CCC, EC, endometrial carcinoma, or uterine cancer of a subject indicates a poor prognosis. The expression level of BAF250a in the tissue sample may be determined in any suitable manner, including for example immunohistochemistry.

In some embodiments, the mutations in BAF250a that indicate a poor prognosis include the mutations set forth in Figure 4.

The presence of mutations in *SMARCA4*, *PBRM1,* or *SMARCC2* that disrupts the function or expression of BRG1, BAF180, or BAF170, respectively, in a sample of tissue obtained from a CCC, EC or uterine cancer of a subject indicates a poor prognosis. The presence of mutations in these genes in the tissue sample can be determined by any suitable method, such as, for example, Sanger sequencing of the tissue sample or next generation sequencing of the tissue sample, PCR-based methods including ARMS-based PCR, FISH, or other suitable detection technique.

The absence of expression of BRG1, BAF180, or BAF170 in a sample of tissue obtained from a CCC, EC or uterine cancer of a subject indicates a poor prognosis. The expression level of BRG1, BAF180 or BAF170 in the tissue sample may be determined in any suitable manner, including for example immunohistochemistry.

The presence of mutations in one or more genes that encode components of the SWI/SNF complex that disrupt the function or expression of the corresponding protein products in a sample of tissue obtained from a cancerous lesion of a subject indicates a low likelihood that treatment of the subject with standard chemotherapeutic agents such as platinum and taxane therapies is likely to be successful. The presence of mutations in such genes can be determined by any suitable method, such as, for example, Sanger sequencing of the tissue sample or next generation sequencing of the tissue sample. In some embodiments of the invention, the one or more genes is *ARID1A*. In some aspects of the disclosure, the one or more genes are *ARID1B*, *ARID2*, *SMARCA2*, *SMARCC1*, *SMARCD1*, *SMARCD2, SMARCD3*, *SCARCE1*, *ACTL6A, ACTL6B,* or *SCMARCB1*.

In some embodiments, the absence of expression of one or more proteins that are components of the SWI/SNF complex in a sample of tissue obtained from a cancerous lesion of a subject indicates a low likelihood that treatment of the subject with standard chemotherapeutic agents such as platinum and taxane therapies is likely to be successful. The expression level of the proteins in the tissue sample may be determined in any suitable manner, including for example immunohistochemistry. In some embodiments of the invention, the one or more proteins includes BAF250a. In some aspects of the disclosure, the proteins are BAF250b, BAF200, BRM, BAF155, BAF60a, BAF60b, BAF60c, BAF57, BAF53a, BAF53b, or BAF47. protein BAF250a, that disrupt the function or expression of BAF250a in a sample of tissue obtained from a CCC, EC, endometrial carcinoma or uterine cancer of a subject indicates a low likelihood that treatment of the subject with standard chemotherapeutic agents such as platinum and taxane therapies is likely to be successful. The presence of mutations in *ARID1A* in the tissue sample can be determined by any suitable method, such as, for example, Sanger sequencing of the tissue sample or next generation sequencing of the tissue sample, PCR-based methods including ARMS-based PCR, or TaqMan^{™} assays, or hybridization-based methods including FISH, or any other suitable detection technique.

In some embodiments, the mutations in *ARID1A* that indicate a low likelihood that treatment of the subject with standard chemotherapeutic agents such as platinum and taxane therapies is likely to be successful include the mutations in SEQ ID NO.:2 through SEQ ID NO.: 122 set forth in Figure 4.

In some embodiments, the absence of expression of BAF250a in a sample of tissue obtained from a CCC, EC, endometrial carcinoma or uterine cancer of a subject indicates a low likelihood that treatment of the subject with standard chemotherapeutic agents such as platinum and taxane therapies is likely to be successful. The expression level of BAF250a in the tissue sample may be determined in any suitable manner, including for example immunohistochemistry.

In some embodiments, the mutations in BAF250a that indicate a low likelihood that treatment of the subject with standard chemotherapeutic agents such as platinum and taxane therapies is likely to be successful include the mutations set forth in Figure 4.

The presence of mutations in *SMARCA4*, *PBRM1*, or *SMARCC2* that disrupts the function or expression of BRG1, BAF180, or BAF170, respectively, in a sample of tissue obtained from a CCC, EC, or uterine cancer of a subject indicates a low likelihood that treatment of the subject with standard chemotherapeutic agents such as platinum and taxane therapies is likely to be successful. The presence of mutations in these genes in the tissue sample can be determined by any suitable method, such as, for example, Sanger sequencing of the tissue sample or next generation sequencing of the tissue sample, PCR-based methods including ARMS-based PCR, or TaqMan^{™} assays, or hybridization-based methods including FISH, or any other suitable detection technique.

The absence of expression of BRG1, BAF180, or BAF170 in a sample of tissue obtained from a CCC, EC, or uterine cancer of a subject indicates a low likelihood that treatment of the subject with standard chemotherapeutic agents such as platinum and taxane therapies is likely to be successful. The expression level of BRG1, BAF180, or BAF170 in the tissue sample may be determined in any suitable manner, including for example immunohistochemistry.

In some embodiments, loss of expression or function of BAF250a is a biomarker for malignancy derived from endometrial epithelium. In some embodiments, *ARID1A* mutation or BAF250a loss is a targetable feature of a cancer. In some embodiments, the cancer is CCC, EC, or uterine cancer.

Mutations in one or more of the genes that encode proteins that are components of the SWI/SNF complex that disrupt the function of the corresponding protein in the SWI/SNF complex may be used in a screen to identify therapeutic targets for treatment of CCC, EC, and/or uterine carcinoma.

Mutations in one or more proteins that are components of the SWI/SNF complex that disrupt the function of that protein in the SWI/SNF complex may be used in a screen to identify therapeutic targets for the treatment of CCC, EC, and/or uterine carcinoma.

The screen used to identify the therapeutic targets may be a synthetic lethal screen. Any suitable cell line that does not express one or more of the SWI/SNF component proteins, expresses one or more of the SWI/SNF component proteins at levels that are too low to maintain proper functioning of the SWI/SNF complex, or a mutant form of one or more of the SWI/SNF component proteins that does not allow proper functioning of the SWI/SNF complex to be maintained, may be used.

The screen may be conducted using 867CL, 867CL-ARID1A-ΔL2007, and 867CL-ARID1A-WT cells. The screen may be conducted using an isogenic knockout of *ARIDIA* in HCT116 cells.

The synthetic lethal screen may use the Hannon/Elledge lenti-shRNA human library. The synthetic lethal screen may use the Dharmacon siGenome pool.

In some aspects of the disclosure, at least one mutation used in the synthetic lethal screen is in the *ARID1A* gene. In some aspects of the disclosure, the at least one mutation in the *ARID1A* gene is one of the mutations in SEQ ID No.:2 through SEQ ID NO.:122. In some aspects of the disclosure, the at least one mutation in the *ARID1A* gene is ARID1A-ΔL2007. In some aspects of disclosure, the at least one mutation in the *ARID1A* gene enclodes a mutant form of the BAF250a protein. In some aspects of the disclosure, the mutant form of the BAF250a protein is one of the mutations set forth in Figure 4.

In some aspects of the disclosure, at least one mutation used in the synthetic lethal screen is in one of the *SMARCA4, PBRM1,* or *SMARCC2* genes. In some aspects of the disclosure, the at least one mutation in the *SMARCA4, PBRM1,* or *SMARCC2* genes is one of the mutations set forth in Figure 5. In some aspects of the disclosure , at least one mutation is in one of the BRG1, BAF180, or BAF170 proteins. In some aspects of the disclosure, the at least one mutation in the BRG1, BAF180, or BAF170 proteins is one of the mutations set forth in Figure 5.

In some aspects of the disclosure, at least one mutation used in the synthetic lethal screen is in one of the *ARID1B, ARID2, SMARCA2, SMARCC1, SMARCD1, SMARCD2, SMARCD3, SMARCE1*, *ACTL6A, ACTL6B,* or *SCMARCB1* genes. In some aspects of the disclosure, at least one mutation is in one of the BAF250b, BAF200, BRM, BAF155, BAF60a, BAF60b, BAF60c, BAF57, BAF53a, BAF53b, or BAF47 proteins.

In some aspects of the disclosure, therapeutic agents are developed to inhibit the activity of one or more targets identified by the synthetic lethal screen. In some aspects of the disclosure such therapeutic agents are used to treat cancers such as CCC, EC, or uterine cancer. In some aspects of the disclosure, treatment involves administering a therapeutically effective amount of the therapeutic agent to the subject in need. Potential therapeutic agents that may be screened against the one or more targets include known drugs, small molecules, natural compounds, chemical libraries, and siRNA.

Reagents for assaying for the presence of a mutation in a gene encoding a protein that forms part of the SWI/SNF complex, including *ARID1A,* or for assaying for expression of a protein that forms part of the SWI/SNF complex, including BAF250a, may be provided in the form of a kit.

Embodiments of the invention are further illustrated with reference to the following examples, which are intended to be illustrative and not limiting.

### Examples

### Example 1.0 - Identification of ARID1A Mutations in Ovarian Carcinomas

Because CCC are genomically stable^{8,9}, it was expected they will have a constricted mutational landscape and recurrent mutations which would be evident from the analysis of a small number of cases.²⁴

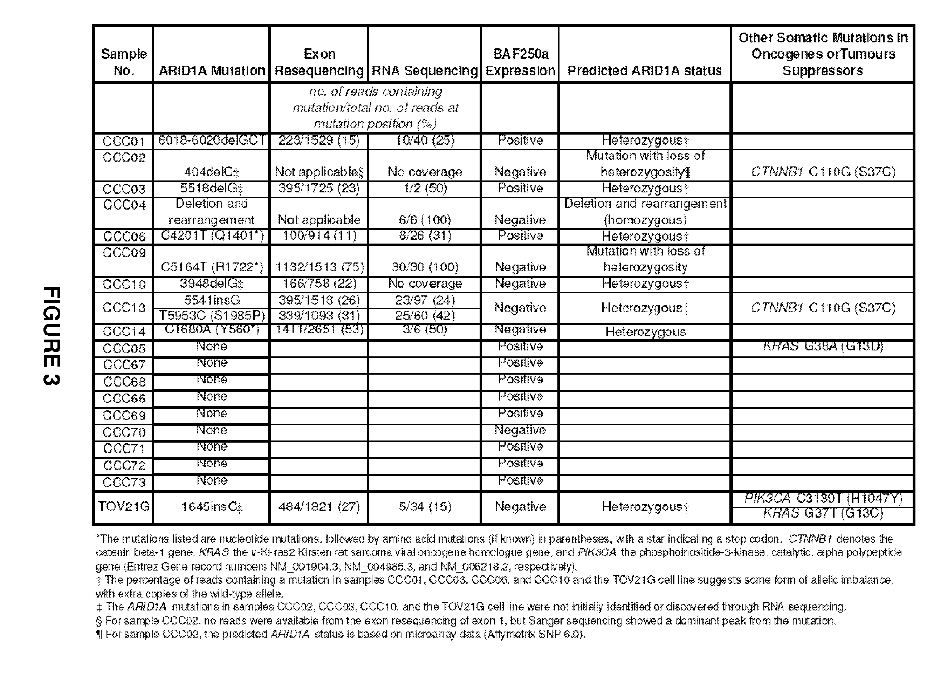

The inventors decoded the transcriptomes of 17 ovarian clear cell cancers using RNA-seq. Gene fusions and small interstitial deletions and insertions were detected by methods described in recent publications^{25,33,34} and SNVs were detected using SNVmix, a Bayesian mixture based algorithm recently published³⁵. The vast majority of SNVs were expected to be rare germline variants as opposed to somatic mutations. Therefore the inventors used the same approach that resulted in identification of the *FOXL2* mutation in granulosa cell tumours¹ to identify genes recurrently mutated in CCCs, but not in unrelated cancer types. The inventors identified mutations in the *ARID1A* gene in six of seventeen CCCs: three cases had nonsense mutations, a fourth case had a 6018-6020delGCT (2007ΔL) 3 base pair deletion mutation, a fifth case had both a somatic missense mutation (T5953C (S1985P)) and a single nucleotide insertion in exon 20 (5541insG), and a sixth case had a genomic deletion spanning intron one resulting in loss of the region 3' to exon 1 in *ARID1A* and fusion to the neighbouring gene (*ZDHHC18*); this was validated by fluorescent in situ hybridization (FISH) (Figures 2 and 3).

All *ARID1A* point mutations were validated by Sanger sequencing, and in all cases where germline DNA was available, mutations were determined to be somatic. Loss of heterozygosity (LOH) was detected in CCC01 which had the 6018-6020delGCT mutation. The *ARID1A* gene was analysed in an additional case of CCC arising in an endometriotic cyst (CCC23) using Sanger sequencing, as this case was not included in the RNA-seq experiments. This resulted in identification of a truncating mutation (G6139T (E2047*)). This case also exhibited LOH through loss of one copy of chromosome 1. Thus, somatic mutations in the *ARID1A* gene were found in seven of eighteen clear cell cancers studied. By comparison, no *ARID1A* mutations were seen in the transcriptomes of 50 triple negative breast cancers, 6 endometrioid, or 6 high grade serous cancers (p=0.00003). A truncating *ARID1A* mutation was found in one of the two mucinous carcinomas of the ovary studied.

With reference to Figure 2, the location of mutations identified by the inventors is shown. BAF250a has a DNA binding or ARID domain (AT-rich interactive domain) of approximately 100 amino acids, and multiple LXXLL (where L is leucine and X is any amino acid) motifs which potentially interact with nuclear hormone receptors. The 20 exons of ARID1A are shown (numbered boxes) above a schematic of the BAF250a protein. In BAF250a, the ARID DNA binding domain ("ARID"), and HIC1 binding domain ("hypermethylated in cancer 1") ("HIC1") are shown. Four LXXLL motifs are indicated and the three C-terminal LXXLL motifs facilitate interaction with glucocorticoid receptor. The nucleotide mutations (with corresponding amino acid mutations in parentheses) listed above the schematic are those identified by means of transcriptome sequencing (RNA sequencing) of the 18 samples of ovarian CCC and the TOV21G cell line. Mutations listed below the schematic are those identified with the use of targeted exon resequencing and Sanger sequencing of genomic DNA from 210 ovarian cancer samples (described below, results shown in Figure 4). All unique somatic mutations detected in samples of ovarian clear-cell carcinoma, endometrioid carcinoma, and high-grade serous carcinoma are shown.

The foregoing results provide strong genetic evidence that *ARID1A,* a gene implicated as a tumour suppressor through functional studies, is frequently disrupted in CCCs.

### Example 2.0 - Identification of Other SWI/SNF Genes Mutated in Ovarian Carcinomas

The inventors have detected SNVs in other SWI/SNF genes including a missense mutation in *SMARCA4* (encodes for BRG1) and a missense mutation in *PBRM1* (encodes BAF180) in *ARID1A*-mutation-negative CCCs (Figure 5). As mutations in *ARID1A* or other SWI/SNF coding genes were found in 9 of 18 (50%) CCCs, these events are important to the development of this cancer. By contrast, mutations in *TP53, BRAF*, *PIK3CA* and *PTEN* were seen in only one, two, two, and three CCCs respectively. This fraction of cases was expected to carry these mutations based on data from previous publications¹⁵.

With reference to Figure 1, the components of the SWI/SNF complex in which mutations were detected are highlighted. All 15 genes encoding protein components of the SWI/SNF complex are shown in the table at left. An example of a BAF250a-containing SWI/SNF complex is shown at right. The arrow indicates that either BAF250a or BAF250b may be in the complex. The PBAF SWI/SNF complex (not shown) has BRG1 and contains BAF200 and BAF180 instead of BAF250a/b. BAF250a encoded by *ARID1A* is implicated in CCCs based on the inventors' mutational data and is shown in orange. Other SWI/SNF genes where the inventors found mutations (*SMARCA4, PBRM1, SMARCC2)* are underlined in the box at left and corresponding proteins are underlined in the cartoon at right (except in the case of BAF180 which is not present in the illustrated complex). Constant core components of the complex are indicated in blue. The ATPase is shown in green.

In addition to variants in *ARID1A*, *CTNNB1* (C110G (S37C), NM_001904.3, SEQ ID NO.: 125) somatic mutations were detected in CCC02 and CCC03 and validated by PCR amplification and Sanger sequencing in both tumor and germline DNA from these cases. Additionally, two variants were predicted based on RNA sequencing data in the TOV21G cell line in *PIK3CA* (C3139T (H1047Y), NM_006218.2, SEQ ID NO.: 123) and *KRAS* (G37T (G13C), NM_004985.3, SEQ ID NO. 124) which were validated by PCR amplification and Sanger sequencing. Though variants in *BRAF* were observed in the RNA sequencing data, none of these passed validation by Sanger sequencing in tumor DNA.

### Example 3.0 Mutations in ARID1A are Associated with Loss of Expression of BAF250a

To demonstrate that *ARIDIA* mutations are associated with loss of expression, the inventors used a mouse monoclonal antibody (Abgent, Inc.) targeting the central region of the BAF250a protein. The antibody stained all normal nuclei strongly. Of the 18 clear cell cancer samples analysed by RNA-seq in Example 1.0, eight showed loss of BAF250a expression. Of these eight cases, five had *ARID1A* mutations (Figure 3). Interestingly, in the other three cases negative for immunohistochemical BAF250a staining, *ARID1A* mutations were not detected by RNA-seq, suggesting that there may be other genetic or epigenetic mechanisms for loss of BAF250a expression. Two cases with *ARID1A* mutations expressed BAF250a; one of these contained an inframe deletion of a single amino acid (6018-6020delGCT (ΔL2007) in CCC01) in exon 20 and the second contained an SNV that created a premature STOP codon (C4201T (Q1401*) in CCC06 in exon 18. The BAF250a expressed in CCC06 may be a truncated protein.

To demonstrate that loss of BAF250a is a subtype-specific finding in ovarian cancer, the inventors stained 300 tumours from their ovarian tumour bank. All non tumour nuclei were strongly positive for BAF250a whereas 11 of 27 CCC cases (40%) showed complete loss of BAF250a in all tumour cells. By comparison, 17 of 180 (10%) high grade serous cancers (p < 0.0001) showed BAF250a loss.

### Example 4.0 - ARID1A Mutation Provides Evidence of Risk of Transformation or Progression of Endometriosis

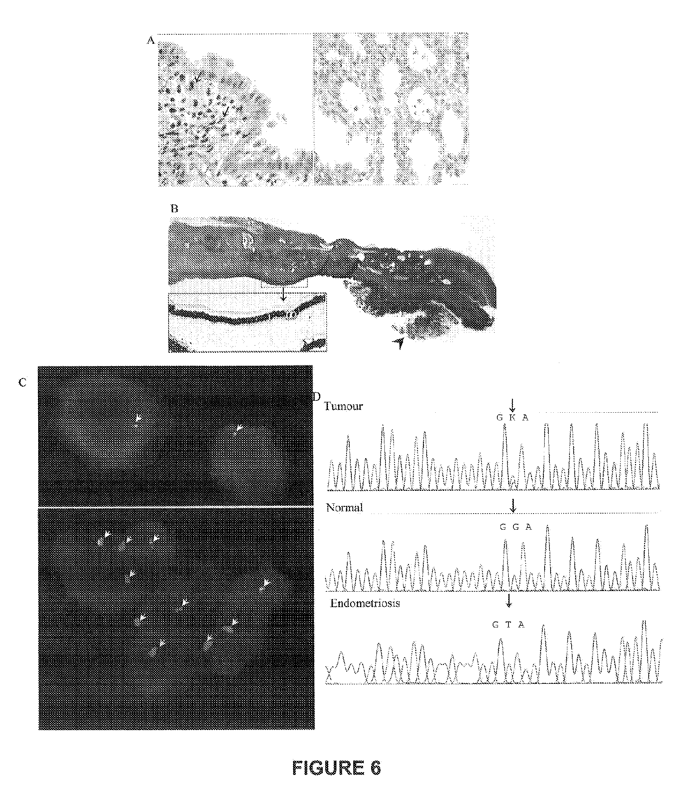

To demonstrate whether *ARID1A* mutations and loss of BAF250a expression are early events in ovarian carcinogenesis, the inventors studied tumour and adjoining endometriosis from case CCC23 which has a truncating mutation in exon 20 and LOH accompanied by complete loss of BAF250a expression (Figure 6). The epithelium but not the stroma of the endometriosis showed loss of BAF250a expression. FISH analysis showed that the endometriosis has LOH at the *ARID1A* locus in a small fraction of cells. Sanger sequencing of cloned PCR products also revealed the mutation in endometriotic epithelial cells. This is the first cancer specific mutation described in endometriosis and suggests that *ARID1A* may play a role in the transformation of endometriosis into cancer.

Figure 6 shows BAF250a expression, *ARID1A* mutations, and loss of heterozygosity in CCC23 and corresponding endometriotic precursor lesions. Panel (A) shows a high magnification view of negative nuclear BAF250a immunostaining from in the endometrial lining of the endometriotic cyst (left) and the clear cell carcinoma arising from the endometrium (right). Normal tissue adjacent to endometriosis is positive for BAF250a expression (arrows). Immunostaining for BAF250a was done with Abgent mouse monoclonal antibody (cat #AT1188a, clone 3H2) diluted 1:25 and run on Ventana Discovery XT with detection by anti-mouse HRP secondary antibody. Panel (B) shows a section of the wall of the endometriotic lesion with area of interest for laser capture microdissection highlighted (red square). Cancerous tissue is indicated by arrowhead (top). Isolated strips of endometrial cells after removal by laser capture microdissection (bottom). Panel (C) shows fluorescent *in situ* hybridization (FISH) analysis of CCC23 tumour (top) which suggests that only a single copy of the *ARID1A* gene is present (arrows), thus there is loss of heterozygosity at the unmutated allele. The red 5' probe (RP11-35M8) was 158,905 bp in length and hybridizes approximately 200 kb upstream of *ARID1A*. The green 3' probe (RP11-285H13) was 183,012 bp in length and hybridizes approximately 130 kb downstream of *ARIDIA.* FISH analysis of endometriosis corresponding to CC23 (bottom) shows a mixture of normal cells with (cell at top left) and cells with loss of heterozygosity at the *ARIDIA* locus (middle and far right cells). Labelling was done using probes flanking *ARID1A* (white arrows) as described along with CEP1 (orange) Vysis centromeric probe (indicated by yellow arrows). Cells with loss of heterozygosity retain two centromeres but have only one copy of the *ARID1A* locus. Panel (D) shows results of Sanger sequencing from CCC23. Mutation (G6139T) and corresponding position in normal tissue is indicated by arrow in tumour, normal, and endometriosis derived samples respectively. Endometriosis sequencing was done using laser microdissection followed by cloning of PCR amplified *ARID1A* into *E. coli.* 48 colonies were sequenced and the mutation was detected in 2 colonies.

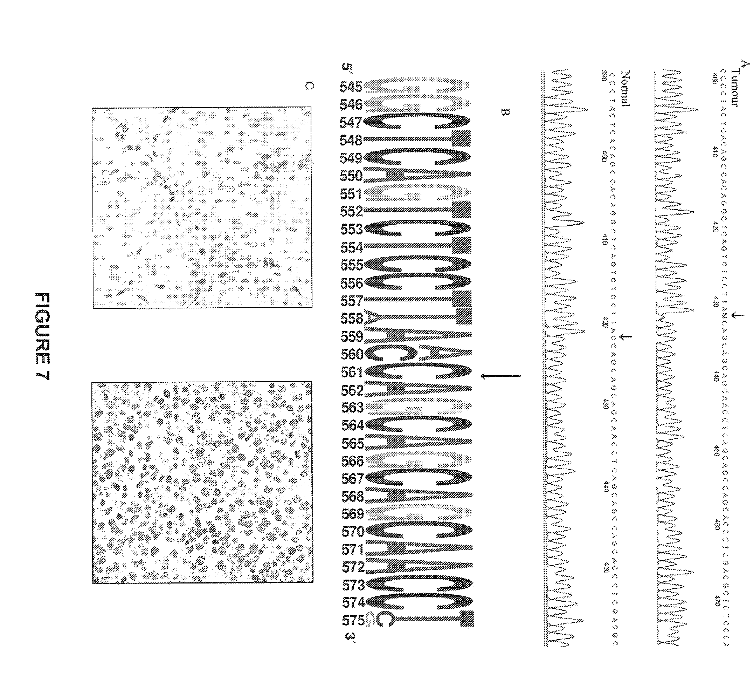

As part of the inventors' tumour banking procedures, they have developed a xenograft sub-renal capsule technique to generate ovarian cancer models in NOD/SCID mice with a greater than 90% rate of successful engraftment to date³⁶. Transplantable xenografts have been established from five clear cell cancers including case VOA867 (CCC14) which has a truncating mutation (C1680A/G, Y560X) accompanied by complete absence of BAF250a protein (Figure 7).

Figure 7 shows the data from case VOA867 (CCC14). Panel (A) shows results from Sanger sequencing of *ARIDIA* from tumour and matched normal DNA. Location of mutation (C1680A) is indicated by arrow. Tumour DNA trace suggests heterozygosity. Panel (B) shows sequence logo from RNA-seq of VOA867 (CCC14) demonstrating that wildtype and mutant alleles are expressed at approximately equivalent frequencies. Mutation (C1680A) is indicated by arrow. Panel (C) shows immunohistochemical staining of BAF250a in VOA867 (CCC14). These results shows lack of expression (left). A non-Hodgkin's lymphoma with positive BAF250a expression is shown at right for comparison. Immunostaining for BAF250a was done using with Abgent mouse monoclonal antibody (cat #AT1188a, clone 3H2) diluted 1:25 and run on Ventana Discovery XT with detection by anti-mouse HRP secondary antibody.

### Example 5.0 - Knock Down of Expression of BAF250a in HCT116 Cells

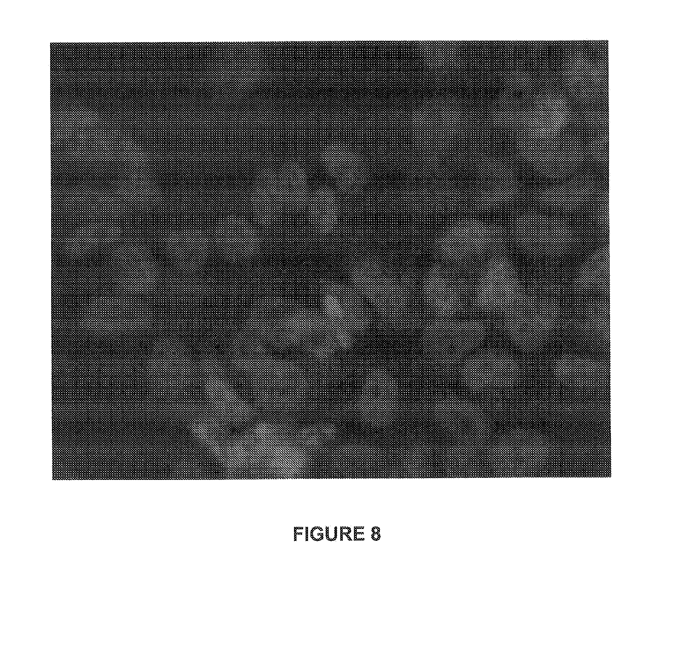

The inventors have also effectively knocked down expression of BAF250a through expression of ARID1A-shRNAmir-GFP in HCT116 cells (Figure 8). Figure 8 shows immunofluorescence data demonstrating knockdown of BAF250a expression through stable expression *ARID1A* shRNA in HCT116 cells. shRNAmir-GFP lentiviral vectors targeting human *ARID1A* sequence (green) (Open Biosystems- shRNA, V2LHS-72862) was packaged and transduced into the human HCT116 colon carcinoma cell line according to the manufacturer's instructions. Well transduced cells with efficient GFP expression show a marked knock down of *ARID1A* (no BAF250a (red) expression) while the non-transduced cells which lack GFP expression stained positive for BAF250a (in red).

### Example 6. 0 - Further Confirmation of The Role of ARID1A in CCC

Based on the results from sequencing the whole transcriptomes of 18 CCCs and a CCC cell line discussed above in Example 1.0, the inventors sequenced *ARID1A* in an additional 210 ovarian carcinomas and a second ovarian CCC cell line. In 2 CCCs, the inventors sequenced DNA from microdissected contiguous atypical endometriotic epithelium to determine whether *ARID1A* mutations were present. The inventors measured BAF250a expression by means of immunohistochemical analysis in an additional 455 ovarian carcinomas.

### Example 6.1 - Materials and Methods

### Example 6.1.1 - Patients and Samples

Eighteen ovarian CCC from the OvCaRe (Ovarian Cancer Research) frozen tumor bank and one CCC cell line (TOV21G) were selected for whole-transcriptome paired-end RNA sequencing. Patients provided written informed consent for research using these tumor samples before undergoing surgery, including acknowledgement that a loss of confidentiality could occur through the use of samples for research. Separate approval from the hospital's institutional review board was obtained to permit the use of these samples for RNA-sequencing experiments.

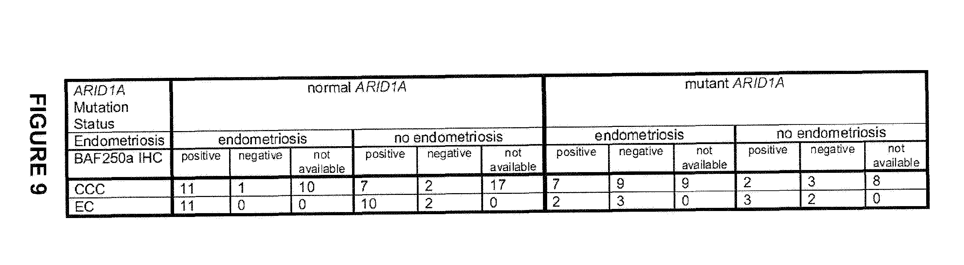

To evaluate the frequency of *ARID1A* mutations in CCC and other ovarian cancer subtypes, the inventors used Illumina based targeted exon resequencing to interrogate the DNA sequence of a mutation validation cohort of 101 CCC (in addition to the 19 cases for RNA seq, described above (the "discovery cohort")), 33 EC, 76 HGS carcinomas and the CCC derived cell line ES2. 10 CCC came from Johns Hopkins University (JHU), 29 from the Université de Montréal (UdeM) and 42 from the Australian Ovarian Cancer Study (AOCS); all other cancers were obtained from the OvCaRe frozen tumor bank. For 70 cases with predicted mutations germline DNA was available. All patients had consented to have their tumors and germline DNA used for research including genomic studies. From the cohort of 119 CCCs (both discovery cohort and mutation validation cohort) and 33 ECs (mutation validation cohort), 86 CCCs and all 33 ECs were examined to determine if endometriosis was present at the time of surgery. These results are shown in Figure 9.

DNA and RNA were extracted using standard methodologies. In cases for which insufficient DNA for *ARID1A* resequencing was available whole genome amplification (WGA) was used to extend the DNA template, however mutations were all confirmed using non-WGA treated DNA.

### Example 6.1.2 - Pathological Review

All tumor samples were independently reviewed by a gynecologic pathologist before mutational analysis. In cases in which the review diagnosis differed from the source diagnosis, the samples were further reviewed by another gynecologic pathologist, who acted as an arbiter. Both review pathologists were unaware of the results of genomic studies.

### Example 6.1.3 - Paired-End RNA Sequencing and Analysis (Whole Transcriptome Sequencing)

Whole transcriptome sequencing was performed as previously described^{1,35}. Double stranded cDNA was synthesized from polyadenylated RNA, and the resulting cDNA was sheared. The 190-210bp DNA fraction was isolated and PCR amplified to generate the sequencing library, as per the Illumina Genome Analyzer paired end library protocol (Illumina Inc., Hayward, CA). The resulting libraries were sequenced on an Illumina GAᵢᵢ . Short read sequences obtained from the Illumina GAᵢᵢ were mapped to the reference human genome (NBCI build 36.1, hg18) plus a database of known exon junctions 2 using MAQ 3 in paired end mode.

Single nucleotide variants were predicted using a Bayesian mixture model, SNVmix^{1,35}. Only bases with > Q20 base quality were considered to minimize errors. SNVs were cross-referenced against dbSNP version 129 and published genomes in order to eliminate any previously described germline variants¹.

Gene fusions were predicted using deFuse. deFuse predicts gene fusions by searching paired end RNA-sequencing data for reads that harbor fusion boundaries. Spanning reads harbor a fusion boundary in the unsequenced region in the middle of the read, whereas split reads harbor a fusion boundary in the sequence of one end. deFuse searches for spanning reads with reads ends that align to different genes. Approximate fusion boundaries implied by spanning reads are then resolved to nucleotide level using dynamic programming based alignment of candidate split reads.

### Example 6.1.4 - Copy Number Analysis of Affymetrix SNP 6. 0 Arrays

The Affymetrix SNP 6.0 arrays were normalized using CRMAv2³⁷ using the default settings for performing allelic-crosstalk calibration, probe sequence effects normalization, probe-level summarization, and PCR fragment length normalization. Log ratios were then computed by normalizing against a reference generated using a normal dataset of 270 HapMap samples obtained from Affymetrix. Segmentation is performed using an 11-state hidden Markov model. This approach simultaneously detects and discriminates somatic and germline DNA copy number changes in cancer genomes. The hidden Markov model performs segmentation of the log ratio intensity data and predicts discrete copy number status for each resulting segment from the set of five somatic states (homozygous deletion, hemizygous deletion, gain, amplification, and high-level amplification), five analogous germline states, and neutral copy number. The boundaries of the segments provide candidate breakpoints in the genome as a result of copy number alteration events.

In all cases with Affymetrix SNP 6.0 data, only CCC04 contained a breakpoint in *ARID1A*. The segment (chr1:26898389-27000523) is a homozygous deletion that breaks the gene near the 5' end and truncates it. The published CNV map from 450 HapMap individuals³⁸ was studied to see whether any regions overlapping *ARID1A* were reported and none were found. Based on this, it is predicted that this is a somatic change.

### Example 6.1.5 - Illumina-based Targeted Exon Resequencing of ARID1A

Genomic DNA for the cases described under *Patients and Samples* above was subjected to Illumina based targeted exon resequencing. Briefly, all *ARID1A* exons were PCR amplified and individual amplicons were indexed, pooled, and sequenced. Individual indexes enabled the deconvolution of reads deriving from individual samples concurrently sequenced from the same library. Validation by Sanger sequencing was performed for all potential truncating or missense mutations with a Grantham index for amino acid change of greater than X, present above a 10% mutant allele frequency cut-off. Insufficient usable data was obtained from exon 1; this was sequenced by Sanger sequencing in all cases using four overlapping amplicons.

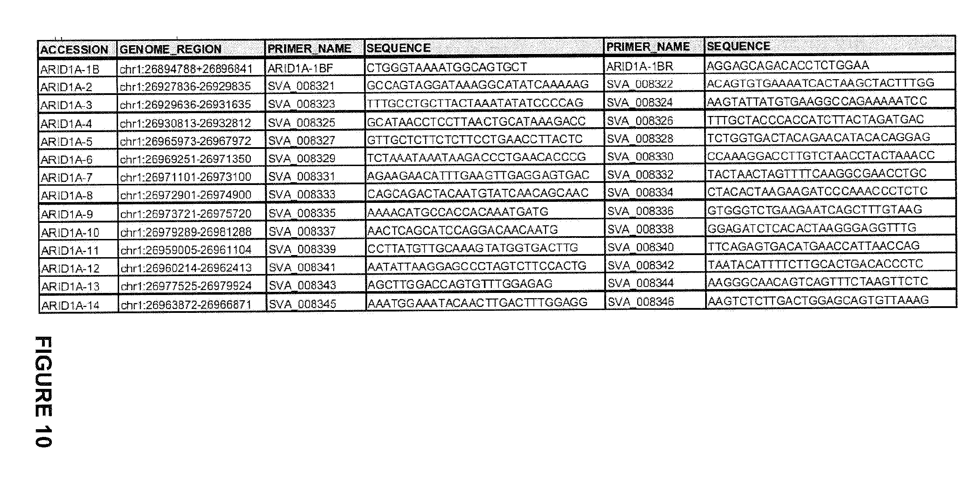

Automated primer design was performed using Primer3³⁹ and custom scripting. Primers were designed to span annotated exons of *ARID1A* (UCSC build hg18) with an average PCR product size of 2067bp. Primers were synthesized by Integrated DNA Technologies at a 25nmol scale with standard desalting (IDT Coralville, IA) and tested in PCR using control human genomic DNA. Primer pairs that failed to generate a product of the expected size were redesigned. The sequences for the primers are provided in Figure 10. Polymerase cycling reactions were set up in 96-well plates and comprised of 0.5 µM forward primer, 0.5 µM reverse primer, 1 ng of gDNA template or 1 ng of gDNA that was whole genome amplified using the REPLI-g^{®} Mini/Midi (QIAGEN, Valencia, CA), 5X Phusion HF Buffer, 0.2 µM dNTPs, 3% DMSO, and 0.4 units of Phusion DNA polymerase (NEB, Ipswich, MA, USA). Reaction plates were cycled on a MJR Peltier Thermocycler (model PTC-225) with cycling conditions of a denaturation step at 98 °C for 30 sec, followed by 35 cycles of [98°C for 10 sec, 69°C for 15 sec, 72°C for 15 sec] and a final extension step at 72°C for 10 min. PCR reactions were visualized by SybrGreen (Life Technologies, Carlsbad, CA, USA) in 1.2% agarose (SeaKem LE, Cambrex, NJ, USA) gels run for 90min at 170V to assess PCR success. Reactions were pooled (4 µl per well) by template and sheared to an average size of 200bp using a Covaris E210 ultrasonic 96 well sonication platform (75 seconds, duty cycle 20, intensity 5, cycles/burst 200; Covaris Inc. Woburn, MA) and subjected to plate based library construction on a BioMek FX Laboratory Automation Workstation (Beckman Coulter, Brea, CA) using a modified paired-end protocol (Illumina, Hayward, CA). This involved end-repair and A-tailing of sheared amplicons followed by ligation to Illumina PE adapters and PCR amplification. At each step in the process, reactions were purified using solid phase reversible immobilization paramagnetic beads (Agencourt AMPure, Beckman Coulter, Brea, CA) in 96 well plates on the BioMek FX platform using custom in house programs. Purified adapter-ligated amplicons were PCR-amplified using Phusion DNA polymerase (NEB, Ipswich, MA) in 10 cycles using PE primer 1.0 (Illumina) and a custom multiplexing PCR Primer [5'CAAGCAGAAGACGGCATACGAGATNNNNNNCGGTCTCGGCATTCCTGCTGA ACCGCTCTTCCGATCT-3'] where "NNNNNN" was replaced with 96 unique fault tolerant hexamer barcodes. Individual amplicons were indexed and pooled by plate and the 200-400bp size range purified away from adapter ligation artifacts on an 8 % Novex TBE PAGE gel (Invitrogen, Carlsbad, CA, USA). Individual indexes enabled the deconvolution of reads deriving from individual samples concurrently sequenced from the same library. DNA quality was assessed and quantified using an Agilent DNA 1000 series II assay (Agilent, Santa Clara CA) and Nanodrop 7500 spectrophotometer (Nanodrop, Wilmington, DE) and subsequently diluted to 10nM. The final concentration was confirmed using a Quant-iT dsDNA HS assay kit and Qubit fluorometer (Invitrogen, Carlsbad, CA). For sequencing, clusters were generated on the Illumina cluster station using v4 cluster reagents and paired-end 75bp reads generated using v4 sequencing reagents on the Illumina GAiix platform following the manufacturer's instructions. Between the paired 75bp reads a third 7 base pair read was performed using the following custom sequencing primer [5'-GATCGGAAGAGCGGTTCAGCAGGAATGCCGAGACCG] to sequence the hexamer barcode. Image analysis, base-calling and error calibration was performed using v1.60 of Illumina's Genome analysis pipeline.

### Example 6.1.6 - Data Processing for ARID1A Illumina-based Targeted Exon Resequencing

Sequence reads from the *ARID1A* targeted exon resequencing experiment were aligned to the genomic regions targeted by the PCR primers using MAQ version 0.7.1. Each exon was assessed for coverage by enumerating all uniquely aligning reads to the targeted space. SNVs were determined by computing the allelic counts for each genomic position within the complete targeted space. All positions exhibiting an allelic ratio of at least 10% variant were considered for validation by Sanger sequencing. Insertions and deletions were predicted using the Maq indelpe program using 10% allelic ratio criteria for selection for experimental follow up. In addition, to determine a confidence measure for each SNV prediction, we applied a one-tailed Binomial exact test to each position covered as described in Shah et al.¹ using all aligned reads to compute the expected distribution. Benjamini-Hochberg⁴⁰ correction for multiple comparison was applied to the resultant Binomial-test p-values to yield q-values for each position.

### Example 6.1.7 - Sanger Sequencing of ARID1A Exon1

The Illumina based targeted exon sequencing of *ARID1A* did not provide coverage of exon 1. To obtain sequence information for exon 1, four overlapping PCR primer sets were designed, priming sites for M13 forward and M13 reverse added to their 5' ends to allow direct Sanger sequencing of amplicons. For the PCR, after denaturation at 94°C for 1 min, DNA was amplified over 35 cycles (94°C 30 sec, 58-60°C 30sec, 72°C 30 sec) using an MJ Research Tetrad (Ramsey, MN). Final extension was at 72°C for 5 min. PCR products were purified using ExoSAP-IT^{®} (USB^{®} Products Affymetrix, Inc., Cleveland, OH) and sequenced using an ABI BigDye terminator v3.1 cycle sequencing kit (Applied Biosystems, Foster City, CA) and an ABI Prism 3130x1 Genetic Analyzer (Applied Biosystems, Foster City, CA). All capillary traces were visually inspected to confirm their presence in tumor and absence from germline traces or analyzed using Mutation Surveyor.

### Example 6.1.8 - Sanger Sequence Validation of Predicted Mutations

Based on the exon resequencing data, any truncating or radical missense mutations (results in change to the charge or polarity of the amino acid⁴¹) that occurred at an allele frequency of greater than 10% were further validated in tumor DNA, and in most cases germline DNA, using Sanger sequencing. Regions of *ARID1A* containing putative mutations were PCR amplified from genomic DNA using primers with priming sites for M13 forward and M13 reverse added to their 5' ends to allow direct Sanger sequencing of amplicons. In cases where the matched germline DNA of the patient was from FFPE material, short (<250nt) amplicons were designed to validate the SNVs.

Unless otherwise stated, amplicons were produced from genomic DNA from both the tumor and matched germline DNA from the same patient. For the PCR, after denaturation at 94°C for 1 min, DNA was amplified over 35 cycles (94°C 30 sec, 60-65°C 30sec, 72°C 30 sec) using an MJ Research (Ramsey, MN) Tetrad. Final extension was at 72°C for 5 min. PCR products were purified using a MinElute PCR purification kit (QIAGEN, Valencia CA) and sequenced using an ABI BigDye terminator v3.1 cycle sequencing kit (Applied Biosystems, Foster City, CA) and an ABI Prism 3130x1 Genetic Analyzer (Applied Biosystems, Foster City, CA). All capillary traces were visually inspected to confirm their presence in tumor and absence from germline traces or analyzed using Mutation Surveyor. Results from this analysis along with immunohistochemistry are summarized in Figure 4.

### Example 6.1.9 - Immunohistochemical Analysis of BAF250a Protein

Immunohistochemical (IHC) staining for BAF250a was performed in all cases with the exception of the 42 CCC from the AOCS and 4 samples from JHU. Additional IHC staining for hepatocyte nuclear factor (HNF)-1β, and estrogen receptor (ER) was performed on whole sections for two cases with associated atypical endometriosis as previously described¹⁴. ER is typically positive in endometriosis and negative in CCC, while HNF-1β is typically negative in endometriosis and positive in CCC¹⁴.

Immunohistochemical analysis was performed on 4 µm thick paraffin sections on the semi-automated Ventana Discovery® XT instrument (Ventana Medical Systems, Tucson, AZ). ARID1A and HNF-1β was stained using the Ventana ChromoMap™ DAB kit. Antigen retrieval was standard CC1 with a two hour primary incubation. ARID1A mouse clone 3H2 (Abgent, San Diego, CA) was applied at 1:25 followed by a 16 minute secondary incubation of pre-diluted UltraMap™ Mouse HRP (Ventana). HNF-1β goat polyclonal (Santa Cruz Biotechnology, Santa Cruz, CA) was applied at 1:200 dilution followed by a 32 minute incubation of unconjugated rabbit antigoat secondary at 1:500 (Jackson ImmunoResearch Labs Inc., West Grove, PA). Afterwards the tertiary antibody was incubated for 16 minutes with the prediluted Ventana UltraMap™ Rabbit HRP. ER immunostaining was done using the Ventana DABMap™ kit with standard CC1. The rabbit clone SP1 (Thermo Scientific, Fremont, CA) was incubated at 1:25 for 60 minutes with heat followed by a 32 minute secondary incubation with the pre-diluted Ventana Universal Secondary. Histologic images were obtained with the use of a ScanScope XT digital scanning system (Aperio Technologies Inc., Vista, CA).

### Example 6.1.10 - Immunohistochemical Analysis of BAF250A - Additional Experiment

A total of 455 additional ovarian-carcinoma samples -- including 132 ovarian clear-cell carcinomas, 125 endometrioid carcinomas, and 198 high-grade serous carcinomas - from a previously described tissue microarray⁴ were used for an immunohistochemical validation cohort and were analyzed for BAF250a expression. All normal gynecologic tissues showed moderate or intense nuclear immunoreactivity for BAF250a. Tumors were scored positive for BAF250a if tumor cells showed definite nuclear staining and negative if tumor nuclei had no immunoreactivity but endothelial and other nontumor cells from the same samples showed immunoreactivity. Cases in which neither normal cells in the stroma nor tumor cells were immunoreactive were considered to be the result of technical failure. Additional immunohistochemical staining for hepatocyte nuclear factor 1β (HNF-1β) and estrogen receptor was performed on whole sections for two tumors with contiguous atypical endometriosis, as previously described.¹⁴

### Example 6.1.11 - Laser Capture Microdissection (LCM), DNA Isolation, and Cloning

In two cases with identified *ARID1A* mutations, atypical (adjacent) and distant endometriosis sections were identified by a gynecological pathologist. Laser capture microdissection was used to isolate endometriotic epithelium. DNA extracted from these cells was analyzed by sequencing for the mutations seen in each case. For microdissection, formalin-fixed paraffinembedded (FFPE) sections (5 µM) were cut on a Tissue-Tek® Cryo3® cryostat (Sakura Finetek, Dublin, OH) onto clean uncharged slides. FFPE sections were deparaffanized and rehydrated, stained with Areturus® HistoGene® Staining Solution (Molecular Devices, Inc., Sunnyvale, CA), then dehydrated in alcohol and xylene. All reagents were prepared with nuclease-free water and all steps were performed using nuclease-free techniques.

Atypical or distant endometriotic cells were microdissected from prepared FFPE sections using the Veritas™ Laser Capture Microdissection System (Arcturus Bioscience, Inc., Mountain View, CA) according to the manufacturer's standard protocols. LCM caps with captured cells were placed directly in 15 µL of lysis buffer with 10 µL of Proteinase K, and DNA was isolated using the QIAamp® DNA Micro kit (QIAGEN, Hilden, Germany). DNA was subsequently quantified on a NanoDrop spectrophotometer (NanoDrop Technologies, Wilmington, DE). PCR was performed, followed by gel extraction of PCR products using the QIAquick Gel Extraction Kit (QIAGEN), PCR products were cloned using the Topo® TA Cloning® Kit following manufacturer's instructions (Invitrogen Corp., Carlsbad, CA). Inserts from individual clones were PCR amplified and Sanger sequenced to determine mutation frequency.

### Example 6.1.12 - Fluorescent In-Situ Hybridization (FISH)

Tissue samples from CCC13 and CCC23 were assayed for deletion of *ARID1A* using fluorescent *in-situ* hybridization (FISH). Six micrometer-thick sections were pre-treated as described previously.⁴² Three-color FISH assays were performed using BACs specific to the regions flanking *ARID1A* (RP11-35M8 (chr1:26,609,021-26,767,926) and RP11-285H13 (chr1:27,033,759-27,216,771)) and fosmids specific to the *ARID1A* locus (G248P86703G10 (chr1:26,976,949-27,017,636), G248P89619A2 (chr1:26,954,143-26,991,761), and G248P88415D8 (chr1:26,914,023-26,954,284)). BAC and fosmid probes were obtained from British Columbia Genome Sciences Centre, and were directly labeled with Spectrum Red, Spectrum Blue, or Spectrum Green using a Nick Translation Kit (Abbott Molecular Laboratories, Abbott Park, Il). Analysis was done on a Zeiss Axioplan epifluorescent microscope. Images were captured using Metasystems Isis FISH imaging software (MetaSystems Group, Inc. Belmont MA). Loss of heterozygosity was confirmed in CCC23 and the results were inconclusive for CCC 13.

### Example 6.1.13 - Gene Expression Analysis

For gene expression analysis, the RNA-sequencing reads initially were mapped to the genome (NCBI36/hg 18) using MAQ (0.7.1). The inventors used the Sequence Alignment/Map (SAMtools 0.1.7) for downstream processing. Up to five mismatches was allowed. Raw expression values (read counts) were obtained by summing the number of reads that mapped to human genes based on the Ensembl database (Release 51). The initial gene expression values were normalized using a quantile normalization procedure using aroma.light (1.16.0.) package in R (2.11.1).

### Example 6.2 - Results

### Example 6.2.1 - ARID1A Mutations

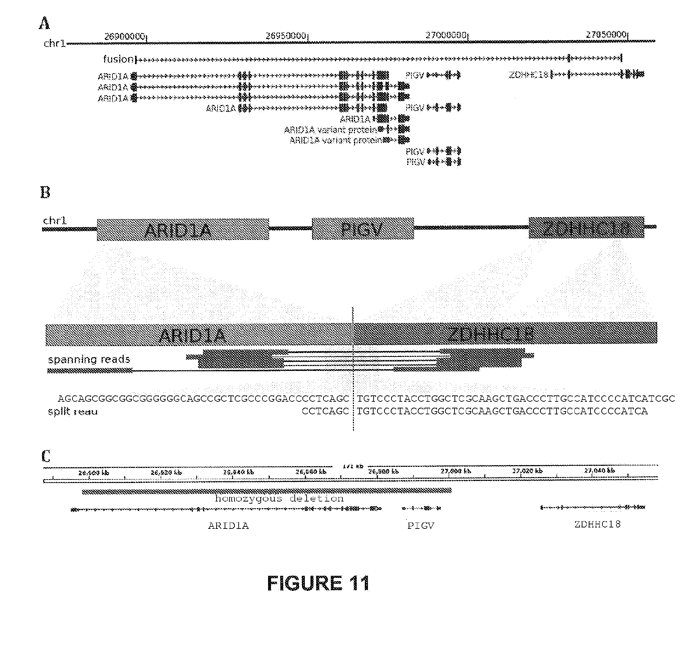

Of the 19 RNAseq samples, 3 had somatic truncating mutations (C4201T (Q1401*), C5164T (R1722*), and C1680A (Y560*), where asterisks denote a stop codon), 2 had somatic indels (insertion-deletion: 6018-6020delGCT and 5541insG), one somatic missense mutation (T5953C (S1989P), found in the same sample as the 5541insG mutation), and 1 had a gene rearrangement involving *ARID1A* and the neighbouring gene *ZDHHC18* encoding the zinc-finger DHHC domain-containing protein 18 (Figures 2 and 3). The fusion ends of this rearrangement map to a homozygous deletion involving most of the *ARID1A* gene which is shown as Figure 11. All predicted variants were validated by Sanger sequencing in DNA from the source tumors. As an exception, the deletion-rearrangement was validated with the use of microarray data (Affymetrix SNP 6.0). These mutations were all somatic.

Since mutations in *PIK3CA* (the phosphoinositide-3-kinase, catalytic, alpha polypeptide gene), *CTNNB1* (the catenin beta-1 gene), KRAS (the v-Ki-ras2 Kirsten rat sarcoma viral oncogene homologue gene), and TP53 (the tumor protein p53 gene) are recurrent in ovarian clear-cell carcinoma,¹⁵ the inventors also analyzed the RNA-sequencing data and performed a polymerase-chain-reaction assay for the presence of variants in these genes (Figure 3). Whole-transcriptome sequence data for the 19 samples of the discovery cohort have been deposited at the European Genome-Phenome Archive (accession number, EGAS00000000075).

*ARID1A* mutation frequency in CCC and other ovarian cancer subtypes was established through Illumina-based targeted exon resequencing of a larger cohort of samples. The total frequency of CCC with significant *ARID1A* mutations is 55/119, or 46%. Only two were somatic missense mutations; the remainder were truncating mutations that were evenly distributed across the coding sequence (Figure 2). *ARID1A* mutations were also commonly seen in EC where 30% (10/33) had confirmed truncating mutations, and in none of the 76 HGS carcinoma with a somatic *ARID1A* missense mutation (mutations summarized in Figure 4). Seventeen cases including 12 CCC and 5 EC each had two validated *ARID1A* mutations.

The inventors analyzed germ-line DNA from 55 samples (47 ovarian clear-cell carcinomas and 8 endometrioid carcinomas) in the discovery and mutation-validation cohorts for the presence of 65 truncating mutations (53 found in ovarian clear-cell carcinomas and 12 found in endometrioid carcinomas). In all 55, the mutations were found to be somatic. On this basis, the inventors made the assumption that 12 subsequent truncating mutations (10 in ovarian clear-cell carcinoma and 2 in endometrioid carcinoma) would be somatic (i.e., predicted to be somatic without germ-line DNA testing) (Figure 4).

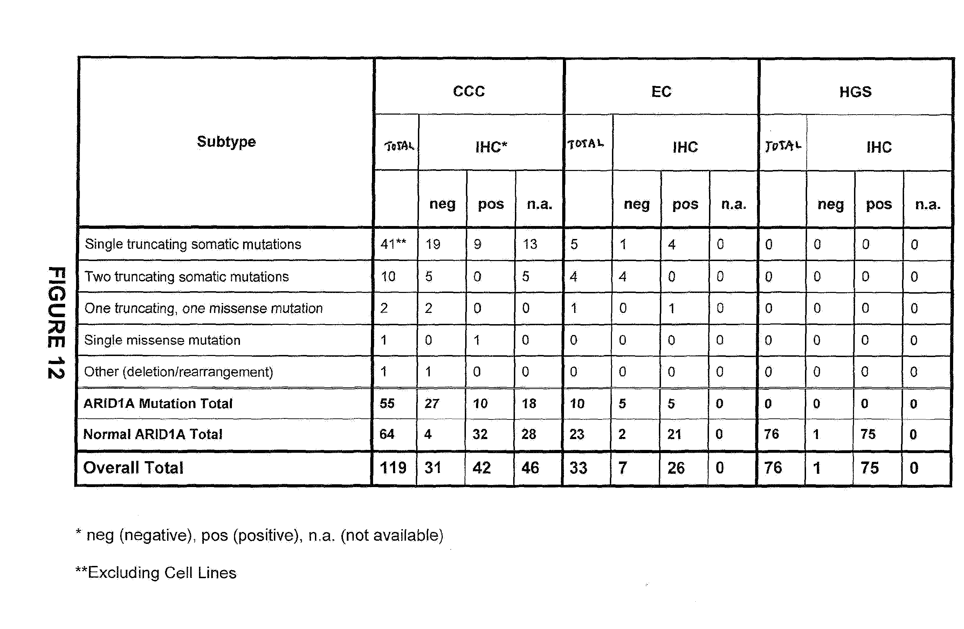

The presence of *ARID1A* mutation shows a strong association (Fisher Exact p < 0.0001) with endometriosis associated ovarian cancer subtypes (CCC or EC) (Figure 12).

### Example 6.2.2 - BAF250a Protein Expression

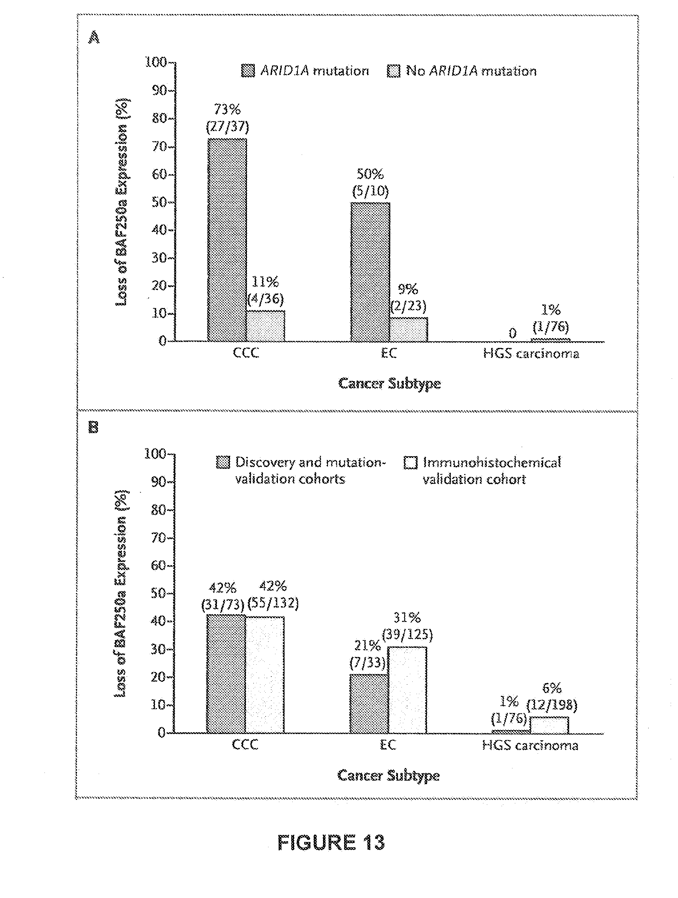

*ARID1A* was further evaluated by IHC staining for BAF250a in 73 CCC, 33 EC and 76 HGS cancers for which formalin-fixed, paraffin-embedded sections were available in the discovery cohort and the mutation-validation cohort. These results are summarized in Figure 13. Loss of BAF250a expression is strongly associated with endometriosis-associated ovarian cancers. In one cohort, 35/74 (47%) of CCC and 7/33 (21 %) of EC but only 1/76 (1 %) of high grade serous cancers showed loss of BAF250a expression (Fisher Exact p=1.70E-10). The presence of truncating mutations in *ARID1A* was significantly associated with BAF250a loss in endometriosis-associated cancers (Fisher Exact p=3.38E-07). Within CCC 27/55 (49%) of cases with truncating mutations showed loss as opposed to 8/35 (23) % of mutation negative cases (see also Figure 4).

In another analysis, the correlation between *ARID1A* mutations and BAF250a expression was evaluated by means of immunohistochemical staining for BAF250a in 182 tumors for which formalin-fixed, paraffinembedded sections were available in the discovery cohort and the mutation-validation cohort described above: 73 ovarian clear-cell carcinomas, 33 endometrioid carcinomas, and 76 high-grade serous carcinomas. The presence of mutations was significantly associated with BAF250a loss in endometriosis-associated cancers (P < 0.001 by Fisher's exact test). A total of 27 of 37 samples (73%) and 5 of 10 samples (50%) of ovarian clear-cell carcinoma and endometrioid carcinoma, respectively, with an *ARID1A* mutation showed a loss of BAF250a expression, as compared with 4 of 36 samples (11%) and 2 of 23 samples (9%), respectively, without an *ARID1A* mutation (Figure 12 and Figure 13A). Loss of BAF250a expression was strongly associated with the endometriosis-related ovarian cancers - with 31 of 73 samples (42%) of ovarian clear-cell carcinoma and 7 of 33 samples (21 %) of endometrioid carcinoma showing a loss of expression - as compared with high-grade serous carcinomas, for which 1 of the 76 samples (1%) had loss of expression (P < 0.001 by Fisher's exact test) (Figure 13A). *ARID1A* mutations were not significantly associated with the presence of endometriosis in 86 ovarian clear cell carcinomas and 33 endometrioid carcinomas (Figure 9).

The immunohistochemical validation cohort was also assessed for BAF250a expression (Figure 13B). This analysis revealed that 55 of the 132 samples (42%) of ovarian clear-cell carcinoma, 39 of the 125 samples (31%) of endometrioid carcinoma, and 12 of the 198 samples (6%) of high-grade serous carcinoma lacked BAF250a expression. These findings are in agreement with the proportions observed in the discovery and mutation-validation cohorts. No significant associations with absence of BAF250a expression were noted on the basis of age of presentation, stage of disease (low or high), or disease-specific survival within any of the cancer subtypes, as assessed by means of Welch's analysis of variance, Fisher's exact test, and the log-rank statistic, respectively (P > 0.05 for all analyses).

With reference to Figure 13, the percentages of tumors (with number and total number in parentheses) from three subtypes of ovarian cancer - clear-cell carcinoma (CCC), endometrioid carcinoma (EC), and high-grade serous (HGS) carcinoma - from the discovery and mutation-validation cohorts that showed loss of BAF250a expression are shown in Panel A for samples with and samples without ARID1A mutations and in Panel B for samples in the discovery and mutation-validation cohorts and samples in the immunohistochemical validation cohort. The rate of BAF250a loss was higher among CCC specimens with an *ARID1A* mutation than among those without an *ARID1A* mutation (P < 0.001); the same was true for EC specimens (P=0.02). The loss of expression was also consistently more common in CCC and EC (the two endometriosis-associated carcinomas) than in HGS carcinoma when assessed in the discovery and mutation-validation cohorts and again in the immunohistochemical validation cohort (Panel B), with P < 0.001 for all comparisons. All P values were calculated with the use of Fisher's exact test.

### Example 6.2.3 - Analysis of ARID1A in Associated Endometriosis

Two patients with ovarian clear-cell carcinomas (samples CCC13 and CCC23) carrying *ARID1A* mutations had contiguous atypical endometriosis.

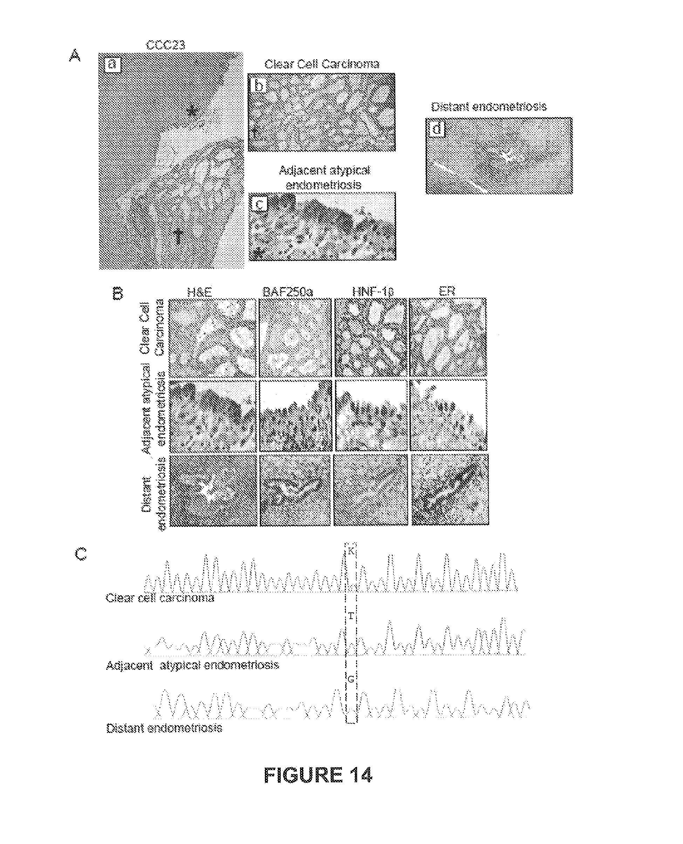

Case CCC23 had an *ARID1A* truncating mutation (G6139T (E2047*)) in exon 20 and had BAF250a loss in both cancer and contiguous atypical endometriotic epithelium (Figure 14); HNF-1β was expressed in the CCC only, and ER was expressed in the atypical endometriotic epithelium. IHC analysis of distant endometriosis, away from the CCC, was also positive for BAF250a and ER expression, and negative for HNF-1β. The E2047* mutation was heterozygous in the tumor and present in 17/42 clones from the contiguous atypical endometriosis and 0/52 clones from a distant endometriotic lesion (Fisher p < 0.0001). Thus, the contiguous atypical endometriosis showed ER expression and absence of HNF-1β expression, similar to distant benign endometriotic lesions, but had the same *ARID1A* mutation as the CCC. Thus, atypical endometrium could be distinguished from the distant endometrium only on the basis of loss of BAF250a expression, which correlated with the presence of an *ARID1A* mutation.

With reference to Figure 14, panel A shows a section (hematoxylin and eosin [H&E]) on which a clear-cell carcinoma (black arrow) has arisen in an endometriotic cyst (white arrow). The same section, viewed at a higher magnification, shows regions of the clear-cell carcinoma and contiguous atypical endometriosis. A region of distant endometriosis from the same patient is also shown. Panel B shows the results of immunohistochemical staining of the epithelial portions of tissue specimens shown in Panel A for expression of BAF250a, hepatocyte nuclear factor 1ß (HNF-1ß), and estrogen receptor (ER). BAF250a immunoreactivity is lost in both the clear-cell carcinoma and the contiguous atypical endometriosis but is maintained in the distant endometriosis. Both regions of endometriosis differ from the carcinoma in their lack of HNF-1ß expression (with weak expression in the contiguous atypical endometriosis) and maintenance of estrogen-receptor expression. Panel C shows sequencing chromatograms for the clear-cell carcinoma and polymerase-chain-reaction (PCR) clones of microdissected material from the contiguous atypical endometriosis and distant endometriosis, from which DNA was extracted. The carcinoma and contiguous atypical endometriosis show nucleotide variation corresponding to G6139T (as indicated with the dashed box); the tumor shows a heterozygous peak at that location, whereas the atypical endometriosis is homozygous for the substitution (in 17 of 42 clones). In contrast, the distant endometriosis shows wild-type sequence (in all 52 clones analyzed). None of the PCR clones from the distant endometriosis showed variation from the wild-type sequence.

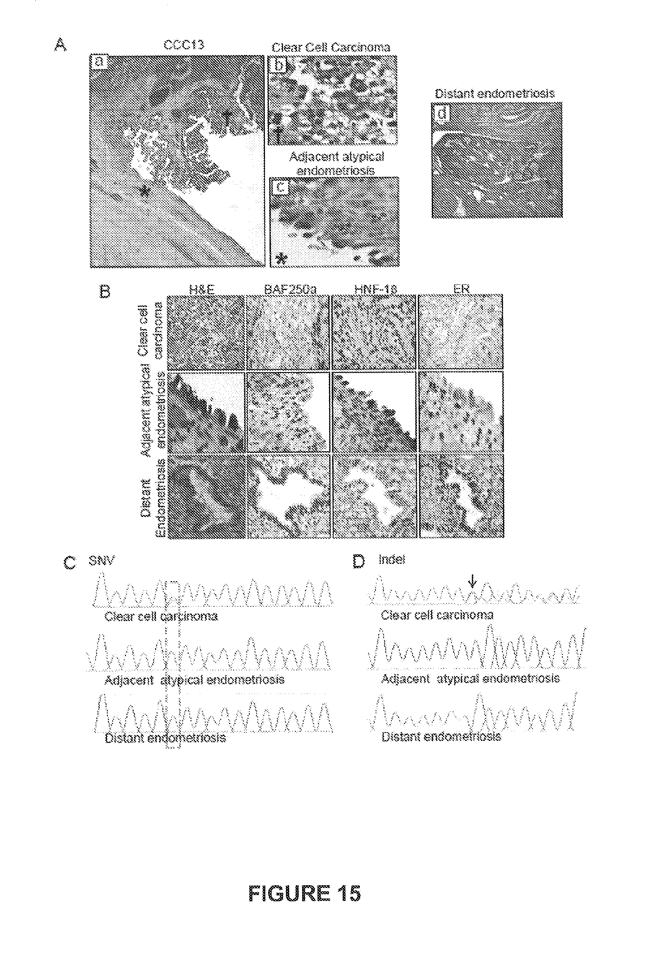

The second case, CCC13, data shown in Figure 15, had two mutations of *ARID1A*: T5953C (S1985P) a somatic missense mutation, and a truncating indel mutation 5541 ins G. Both mutations were heterozygous in the tumor and all cloned PCR products from distant endometriosis were negative for the mutations (0/58 for T5953C; 0/59 5541InsG). In contrast, the missense mutation was present in 20/51 clones from the adjacent atypical endometriosis whereas the indel mutation was seen in only 3/54 clones supporting that this insertion may be a second hit involved in the clonal evolution of the endometriosis into the CCC. Both these mutations, along with a *CTNNB1* missense mutation, were present in the tumor and the adjacent atypical endometriosis but not in a distant endometriotic lesion (Figure 15, panel B).

With reference to Figure 15, results for clear cell carcinoma and adjacent atypical endometriosis for specimen CCC 13 are shown. Panel A shows H&E stained sections from clear cell carcinoma (*) arising in an endometriotic cyst (†) at low power showing adjacent histologies (a), and at higher power showing regions of the clear cell carcinoma (b) and adjacent atypical endometriosis (c). A distant region of endometriosis from the same individual is shown at low power (d). Panel B shows that BAF250a immunoreactivity is lost in the epithelial portion of both the clear cell carcinoma and adjacent atypical endometriosis, however is maintained in the distant endometriosis. HNF-1β can be seen in both the tumor and the adjacent atypical endometriosis, however is largely negative with only occasionally positive cells in the distant endometriosis. ER is highly expressed only in the distant endometriosis and is lost in both the tumor sample and adjacent atypical endometriosis. Panel C shows sequencing chromatograms from the clear cell carcinoma and a PCR clone from contiguous atypical endometriosis clearly show the nucleotide variation corresponding to T5953C (S1985P). This mutation was present in 20/51 clones from the contiguous atypical endometriosis. In contrast, all cloned PCR products (from 58 clones) from distant endometriosis, which maintained BAF250a expression, show only wild type sequence. A heterozygous peak is seen in the DNA from the tumor. Micro-dissected material from both endometriosis samples was used to extract DNA, amplify by PCR, clone and sequence. None of the PCR clones from the distant endometriosis showed variation from the wild-type sequence. Panel D: as in panel "C" sequencing chromatograms from the clear cell carcinoma and a PCR clone from contiguous atypical endometriosis show an insertion of an additional G (5541InsG). This mutation was present in 3/54 clones from the contiguous atypical endometriosis. In contrast, all cloned PCR products (from 59 clones) from the distant endometriosis, which maintained BAF250a expression, show only wild type sequence. Sequencing read from the tumor sample shows characteristic overlapping reads corresponding to the in frame and out of frame alleles after the insertion point. As in "C" sequence from PCR clones are shown for both adjacent atypical endometriosis and distant endometriosis.

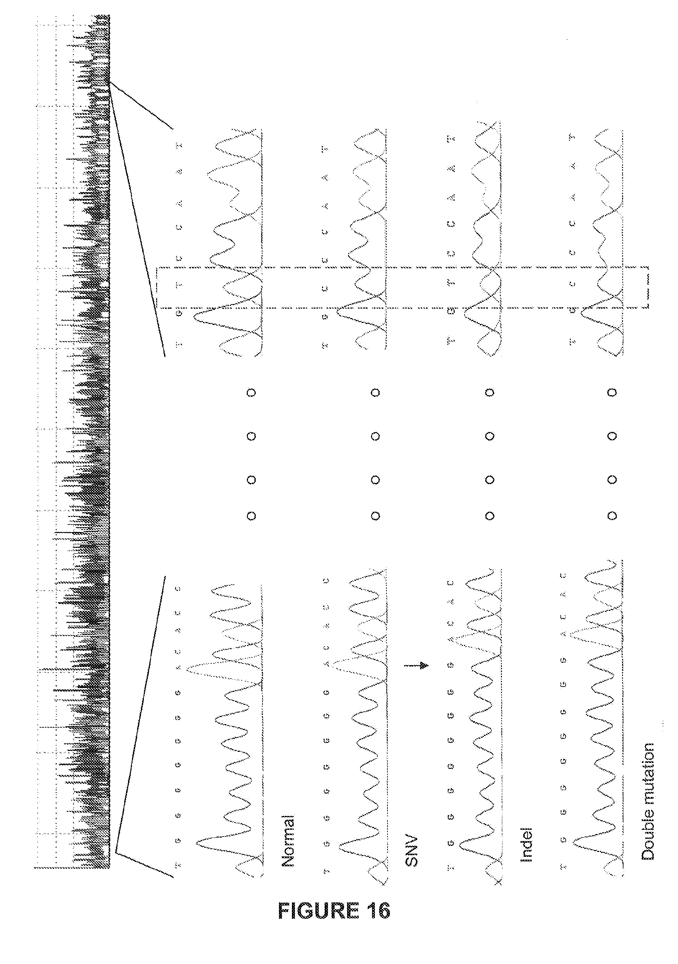

Sanger sequencing was carried out on CCC13. The two somatic mutations (5541insG and T5953C (S1985P)) were sequenced from a single PCR fragment. PCR products were cloned and then resequenced. In total, sequences from 45 clones were analyzed. The inventors found 15/45 (33%) wildtype sequence, 9/45 (20%) sequences with the T5953C (S1985P) mutation, 9/45 (20%) sequences with the 5541insG mutation, and 12/45 (27%) sequences with both mutations in a single Sanger sequence trace. This reveals the complex relationship between the mutations which occur both in trans (on independent alleles) and also in cis (on the same allele) (see Figure 16). This finding along with the presence of wildtype alleles, suggest that this tumor is aneuploid and a gene conversion or other rearrangement at the *ARID1A* locus has occurred and is present in a subset of cells.

Mutations including truncating and somatic missense mutations, and one *ARID1A* rearrangement, were seen in 56/119 (47%) CCCs and 10/33 (30%) ECs (66/153 or 43% in total); but in only 1/76 (1 %) high-grade serous ovarian carcinomas. All truncating mutations for which germline DNA was available were somatic and fifteen cases had two somatic mutations. Loss of BAF250a protein correlated strongly with truncating mutations. In two CCCs the *ARID1A* mutations and loss of BAF250a expression was evident in the tumor and contiguous atypical endometriosis, but not in distant endometriotic lesions or normal tissue.

### Example 6.2.4 - Differential Gene Expression in ARID1A Mutants

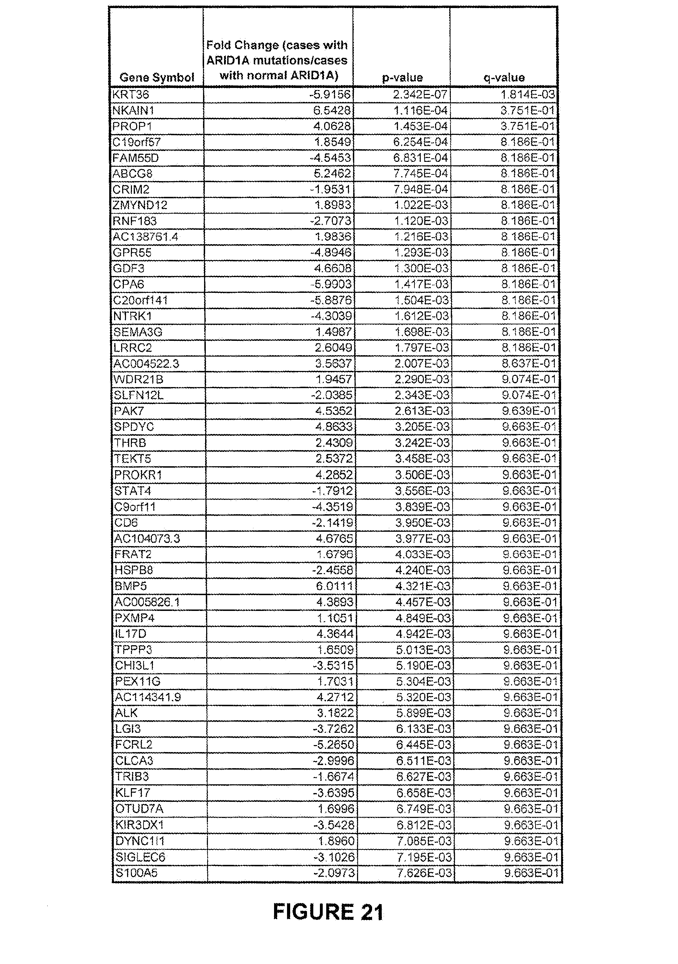

Results for the 50 genes with the greatest differential expression with respect to cells having an *ARID1A* mutation are shown in Figure 21. Figure 21 shows both the genes differentially expressed in mutant *ARID1A* containing cells versus wild-type, and the fold-change in expression of these genes relative to wild type. These genes represent potential target genes to be used in synthetic lethal screening, and also represent potential drug targets for development of new CCC, EOC, uterine cancer treatments.

### Example - 6.3 Discussion of Experimental Results

Overall, 46% of CCC and 30% of EC had somatic truncating or missense mutations in ARID1A as opposed to none in 76 specimens of HGS carcinoma analyzed. Loss of *ARID1A* expression was also subtype specific with loss of nuclear BAF250a seen in 39% of CCC and EC but only 1 % of HGS carcinomas.

There are a number of lines of evidence supporting a significant biological role for somatic *ARID1A* mutations. Firstly, the mutations identified are almost exclusively truncating mutations, expected to encode non-functional protein. They are present at a high frequency in endometriosis associated ovarian carcinomas but not HGS carcinoma, two distinct tumor types, strongly suggesting that they are highly relevant in the former, and not random events. By comparing clear cell carcinomas to their adjacent atypical endometriotic lesions, the inventors have demonstrated that the same mutations are present in the putative precursor lesions as the tumors. In contrast, the distant endometriotic lesions are mutation negative.

In the case shown in Figure 14, the mutation is present before the atypical endometriosis has developed the immunophenoptype associated with the cancer (ER negative, HNF-1β positive) suggesting that the mutation is a very early event in neoplastic transformation. The presence of mutations is strongly correlated with loss of BAF250a protein, suggesting that the normal allele is usually lost, and further supporting an important role for *ARID1A* in oncogenesis. Lastly the finding of two mutation events at the locus in 15 cases, together with the finding of truncating mutations spread evenly across the coding region and frequent loss of protein expression, suggests that *ARID1A* is a classic tumor suppressor gene. Unlike BRCA or p53 mutations, which can be found in the germline, all *ARID1A* mutations were somatic; this may be explained by the observation that heterozygous mutation of *ARID1A*.*is* an embryonic lethal mutation in mice.

Four additional mutations were identified when the RNAseq cases were analyzed by amplicon exon resequencing; these mutations were likely not seen in RNAseq data due to transcripts being rapidly targeted for nonsense mediated decay (NMD)⁴³, indicating that RNAseq, although a useful discovery tool, has imperfect sensitivity for detecting nonsense and other truncating mutations.

In CCC and EC loss of expression was seen in 67% of mutation positive cases and only 16% of mutation negative cases. It is possible that the mutant negative CCC and EC with loss of BAF250a expression may have lost *ARID1A* expression through other mechanisms such as chromosomal rearrangements, epigenetic silencing, expression of transcriptional repressors or post-translational mechanisms. The presence of BAF250a immunoreactivity in a minority of cases with protein truncating mutations may indicate that haploinsufficiency (which is embryonic lethal in a mice) is pathogenic. Alternatively it may be due to second hit events that do not impact protein expression levels, a dominant negative function of some mutations, or detection of truncated but dysfunctional protein in the IHC assay. The latter is possible in some cases as the antibody used targets the middle of the protein (between exons 14-16).

Though there is long standing evidence that endometriosis is a major risk factor for CCC and EC, the molecular mechanism of this transformation is unknown^{44,45}. Mutations in the PTEN gene have been described in 20% of endometriotic cysts. In a mouse model, Cre-mediated expression of oncogenic K-ras was found to induce endometriosis, while a second hit in the tumor suppressor Pten caused progression to endometrioid carcinoma, however K-ras mutations are not seen in human endometriosis or endometriosis associated ovarian cancers.

Gaining an understanding of initiating events for CCC and EC subtypes could lead to the development of new therapeutic approaches and enable the creation of identification tools for endometriotic lesions that are at risk for neoplastic transformation. Mutations in *ARID1A* and loss of BAF250a expression were preferentially seen in CCC and EC, cancers that do not feature the genomic chaos, near ubiquitous TP53 mutations, and frequent BRCA abnormalities of HGS carcinomas. If HGS carcinomas are characterized by gross structural abnormalities in chromosomes, it is possible that defects in genes that alter the use of chromatin, along with previously described WNT and PI3 kinase pathway mutations will define CCC and EC. If such a model is correct, other abnormalities impacting the *ARIDIA* locus or dysregulation of other chromatin remodeling genes will be found in the *ARID1A* mutation negative CCC and EC. This is supported by the clinical similarities between ovarian clear-cell carcinomas positive for and those negative for an *ARID1A* mutation.

The mechanism by which somatic mutations in *ARID1A* enables the progression of the benign condition of endometriosis to carcinoma has yet to be elucidated, however, the foregoing findings strongly suggest a fundamental role for *ARID1A* mutation in the genesis of both CCC and EC. The loss of *ARID1A* in endometriotic epithelium appears to be of importance in malignant transformation in this tissue type.

These data implicate *ARID1A* as a tumor suppressor gene frequently disrupted in CCC and EC. As *ARID1A* mutation and loss of BAF250a can be seen in the pre-neoplastic lesions, this is an early event and likely critical in the transformation of endometriosis into cancer.

### Example 7. 0 - Loss of BAF250a Expression is Common in Endometrial Carcinomas but Infrequent in Other Types of Malignancies

To demonstrate whether BAF250a loss is common in other malignancies, immunohistochemistry (IHC) screening for BAF250a expression was performed on tissue microarrays (TMAs) in more than 3000 cancers, including carcinomas of breast, lung, thyroid, endometrium, kidney, stomach, oral cavity, cervix, pancreas, colon, and rectum, as well as endometrial stromal sarcomas, gastrointestinal stromal tumours (GIST), sex cord-stromal tumours and four major types of lymphoma (diffuse large B-cell lymphoma [DLBCL], primary mediastinal B-cell lymphoma [PMBCL], mantle cell lymphoma [MCL], and follicular lymphoma). The inventors have demonstrated that BAF250a loss is frequent in endometrial carcinomas, but infrequent in other types of malignancies, with loss observed in 29% of Grade 1 or 2, and 39% of Grade 3 endometrioid carcinomas of the endometrium, 18% of high grade serous, and 26 % of clear cell carcinomas. Since endometrial cancers showed BAF250a loss, the inventors stained whole tissue sections for BAF250a expression in 9 cases of atypical hyperplasia and 10 cases of atypical endometriosis. Of the 9 cases of complex atypical endometrial hyperplasia, all showed BAF250a expression, however of 10 cases of atypical endometriosis (the putative precursor lesion for clear cell and ovarian carcinoma), one case showed loss of staining for BAF250a in the atypical areas with retention of staining in areas of non-atypical endometriosis; this was the sole case that recurred as an endometrioid carcinoma, indicating that BAF250a loss may be an early event in carcinogenesis. Since BAF250a loss is seen in endometrial carcinomas at a rate similar to that seen in ovarian carcinomas of clear cell and endometrioid type and is uncommon in other malignancies, loss of BAF250a is a particular feature of carcinomas arising from endometrial glandular epithelium.

### Example 7.1 - Materials and Methods

### Example 7.1.1 - Sample Collection

Cases from the archives of Vancouver General Hospital, St. Paul's Hospital, and the British Columbia Cancer Agency were used to construct tissue microarrays (TMA) from duplicate 0.6 mm cores, as described previously⁴⁶. The follicular lymphoma TMA was constructed using duplicate 1.0 mm cores. For the studies of atypical hyperplasia of the endometrium, hysterectomy cases where there was no co-existent carcinoma were used and full sections were immunostained. Immunostaining on the cases of atypical endometriosis was also performed on full sections. All prospectively collected patient samples were collected with informed patient consent under a research ethics board (REB)-approved protocol, and analysis of archived samples was covered by pre-existing REB approvals.

### Example 7.1.2 - Immunohistochemical (IHC) staining

Immunohistochemical (IHC) staining for BAF250a was performed on all cases included in this study. IHC was performed on 4 µm thick paraffin sections of tissue microarrays or whole tissue sections on the semi-automated Ventana Discovery^{®} XT instrument (Ventana Medical Systems, Tucson, AZ) using the Ventana ChromoMap™ DAB kit. Antigen retrieval was standard CC1 with a two hour primary incubation. BAF250a mouse clone 3H2 (Abgent, San Diego, CA) was applied at 1:50 followed by a 16-minute secondary incubation of pre-diluted UltraMap™ Mouse HRP (Ventana). Histologic images were obtained with the use of a ScanScope XT digital scanning system (Aperio Technologies Inc.,Vista, CA).

### Example 7.1.3 - IHC Scoring

The scoring for BAF250a was performed as previously described⁴⁷. Non-neoplastic cells, including endothelial cells, fibroblasts, and lymphocytes, normally show BAF250a nuclear staining and served as positive internal controls. Positively scored tissue cores were ones that contained any positive tumour cell nuclear staining, regardless of intensity. Negatively scored tissue cores were ones that showed completely absent tumour cell nuclear staining, as well as positive nonneoplastic cell nuclear staining. Tissue cores lacking tumour cells were not scored. Cases in which neither normal cells in the stroma nor tumour cells were immunoreactive were considered to be the result of technical failure. Each case on a tissue microarray was represented as duplicate cores; one positive core in a duplicate was sufficient to count the case as positive.

### Example 7.2 - Results

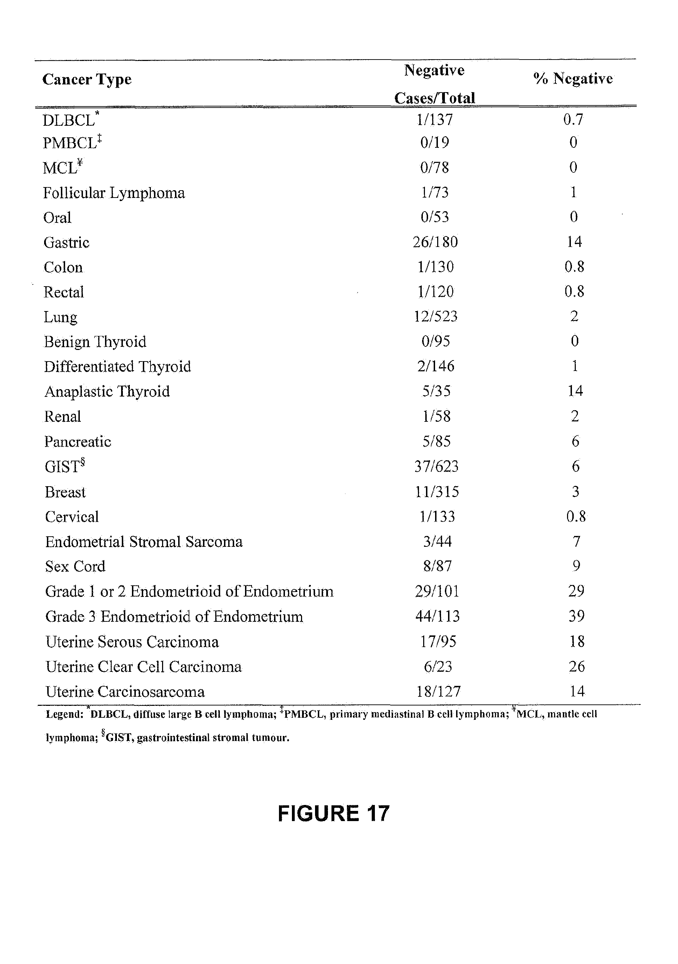

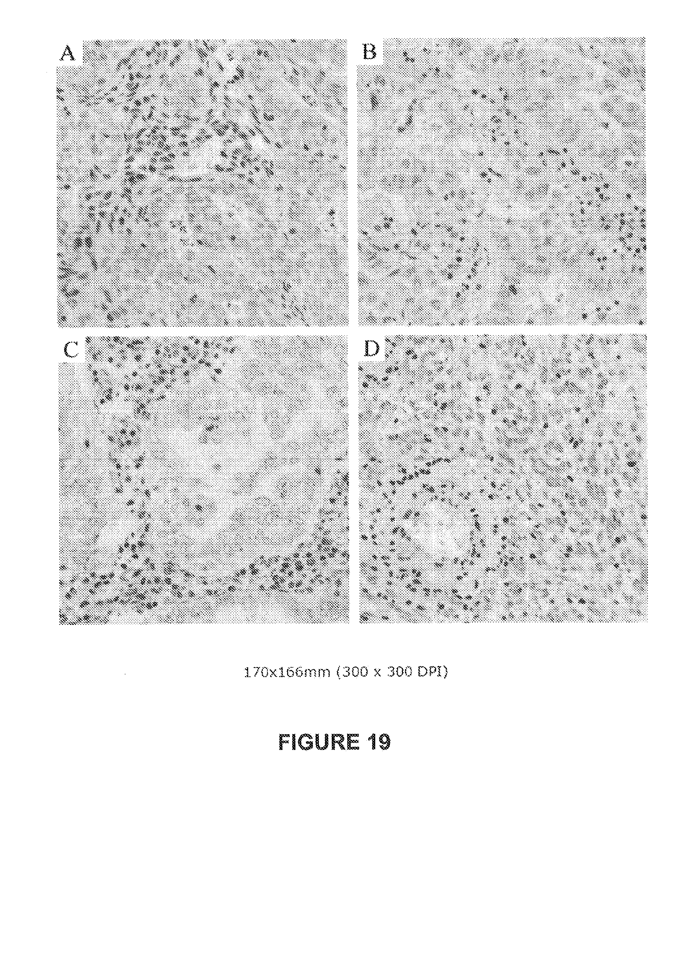

Overall, loss of BAF250a expression measured by IHC was not a common event in nongynaecological malignancies (Figures 17 and 18), with loss of BAF250a in more than 10% of cases of a given tumour type only seen in gastric cancer (14%) and anaplastic thyroid carcinoma (14%). Cancers of endometrial origin showed the highest frequency of BAF250a loss, with 29% of Grade 1 or 2 endometrioid, 39% of Grade 3 endometrioid, 26% of clear cell, and 18% of high grade serous cancers of the endometrium showing BAF250a expression loss (Figures 17 and 19), while 14% of uterine carcinosarcomas showed BAF250a loss.

Nine cases of complex atypical hyperplasia of the endometrium were stained for BAF250a, and all nine showed the same pattern of staining as adjacent normal endometrium (i.e. moderate to intense nuclear positivity). Of the ten cases of atypical endometriosis, all but one showed retention of BAF250a (i.e. normal staining pattern). A single case showed of loss of staining in the cytologically atypical areas with retention of staining in non-atypical endometriosis (Figure 20). This patient developed frank carcinoma of endometrioid type at this site (cul-de-sac) 2 years later.

### Example 7.3 - Discussion

BAF250a, the protein encoded by *ARID1A* (the AT-rich interactive domain1A gene) is one of the accessory subunits of the SWI/SNF chromatin remodeling complex believed to confer specificity in the regulation of gene expression^{27,28}. The SWI/SNF complex consists of multiple components, with the core catalytic subunit utilizing ATP to mobilize nucleosomes, thus providing transcriptional control of genes by altering the accessibility of the promoter regions by the transcriptional machinery. The SWI/SNF complex, ubiquitous in eukaryotes, is important for the regulation of diverse cellular processes, from development, differentiation and proliferation to DNA repair and tumour suppression²⁶.

The results of this Example establish that loss of BAF250a is characteristic of a wide range of tumours arising from eutopic as well as ectopic endometrium, but is uncommon in other tumour types studied. The carcinomas of the endometrium, particularly those of higher grade, show the most frequent loss of BAF250a. In the carcinomas of the endometrium that showed BAF250a loss, the mutational status of the *ARID1A* gene is not known. However in the clear cell and endometrioid carcinomas of the ovary, mutation of *ARID1A* correlates well, although not perfectly, with BAF250a expression. Therefore, the inventors hypothesize that in carcinomas of the endometrium with BAF250a loss, most will harbor mutations in the *ARID1A* gene. In cases that do not show BAF250a loss, it is possible that other components of the SWI/SNF chromatin remodeling complex will show loss of function. Additionally, since the deletion of *ARID1A* on one allele results in embryonic lethality in mice, it is possible that mutations in *ARID1A* resulting in partial loss of BAF250a expression could have a biologic effect in tumours and the effect of *ARID1A* may be underestimated by screening for total BAF250a loss by IHC⁴⁸. The measurement of partial loss would require a nuanced approach to scoring or the use of multiplexed immunofluorescence.

In this study, the inventors did not identify BAF250a loss in any of the nine cases of atypical endometrial hyperplasia. One of the ten cases of atypical endometriosis had loss of BAF250a expression. This patient returned two years later with an endometrioid carcinoma at the location of the atypical endometriosis. This finding could be interpreted in two ways. Firstly BAF250a loss and thus *ARID1A* mutation is a late event in the progression of precursor lesions to cancer or that the particular lesion studied was already fully malignant, although not recognized as such on morphological grounds. Either way, this case along with the frequency of BAF250a loss in frank carcinomas, the rarity (or absence) of loss in normal tissue and precursor lesions suggest that loss of BAF250a expression is a feature highly indicative of malignancy.

### Example 8.0 - Prospective Examples

### Example 8.1 - Demonstrate the Frequency and Clinical Significance of ARID1A and other SWI/SNF Mutations in Ovarian Carcinoma Subtypes

Approximately 30 genes including all 15 SWI/SNF genes will be analyzed for mutations in 150 clear cell carcinomas and 350 other ovarian cancers, using targeted next generation sequencing. When available, precursor lesions will be analyzed to assess if SWI/SNF mutations are early events in oncogenesis. It is predicted that tumours with SWI/SNF mutations will not contain mutations affecting pathways known to drive type I ovarian cancers, so samples will also be analysed for mutations in selected genes associated with these pathways. The 400 cases analysed by targeted resequencing along with an additional 1500 ovarian cases (that have clinical outcome data) will be immunohistochemically analysed to identify cases with loss of BAF250a expression and determine whether this correlates with *ARID1A* mutation status.

As described above, the inventors have demonstrated that approximately 39% of CCCs harbour mutations in the *ARID1A* gene. An additional two cases had mutations in other SWI/SNF complex genes. This observation will be expanded to determine the frequency of mutations in *ARID1A* and the other 15 genes coding SWI/SNF complex proteins mutations in a large cohort (~ 400 cases) of ovarian carcinomas, including all pathological subtypes of this disease,^{26,49} to determine how frequently this complex is perturbed in ovarian cancer.

It is predicted that alterations in the SWI/SNF complex represent a mechanism of oncogenesis of fundamental significance, distinct from previously identified molecular pathways in ovarian carcinoma. This prediction will be confirmed by assessing the mutational status of several genes that are known to be involved in ovarian carcinomas. It is anticipated that chromosomally stable type I ovarian cancers will be able to be sub-categorized into two groups: (i) cancers with mutations in known oncogenic pathways and (ii) cancers with mutations affecting chromatin remodelling. Immunohistochemistry will be used to assess BAF250a expression in the 400 sequenced cases along with 1500 additional ovarian cases.

DNA from 400 frozen ovarian tumour samples representing all subtypes will be used for targeted resequencing. All cases will have an accompanying source of germline DNA. Approximately 150 of these samples will be CCCs and the remaining 250 will be comprised of other ovarian cancer subtypes (50 endometrioid, 150 high grade serous, 25 low grade serous and serous borderline, and 25 mucinous and mucinous borderline). All 250 tumours representing non-CCC subtypes plus 35 CCCs will be obtained from the OvCaRe Tissue Bank (http://www.ovcare.ca/research/platforms.php) located in the Department of Pathology at the Vancouver General Hospital. The remaining 115 CCCs will be obtained from outside sources, such as 42 CCCs from the Australian Ovarian Cancer Study, 30 CCCs from the Institut du cancer de Montréal, 33 CCCs from Mt. Sinai School of Medicine, New York, 10 CCCs from Johns Hopkins University, and 9 CCC cell lines from Dr. Michael Anglesio. With 150 CCC cases, the rate of mutations in CCC will be determined with a margin of error of 8% or less (95% confidence level).

For immunohistochemical analysis of BAF250a protein expression, in addition to the 400 samples described above, another 1500 ovarian cancer samples assembled into tissue microarrays will be examined. These tissue microarrays include approximately 250 CCCs with the remaining cases representing other ovarian cancer subtypes, and have been described previously.^{4,50} In addition, 50 putative CCC precursor lesions, i.e. endometriosis and atypical endometriosis, will be analysed. Lesions from tumours used for targeted sequencing, described above, will be prioritized and the remaining cases will be from the Vancouver General Hospital Pathology Archives.

The 15 SWI/SNF genes along with genes known to be mutated in ovarian cancer including *TP53, KRAS, BRAF, PTEN, PI3KCA, CTNNB1*, *BRCA1*, and *BRCA2* will be sequenced. In total these include 406 exons and intron exon boundary sequence covering 120 kb. To accomplish this, genomic DNA libraries will be enriched with target genes, which will be analysed by next generation sequencing. Alternative approaches are less attractive as high throughput Sanger sequencing is expensive and insensitive to mutations found in less than 15% of alleles due to stromal contamination or intra-tumoural heterogeneity, sequencing of the polyA + transcriptome would not detect mutations resulting in nonsense mediated mRNA decay, and whole exome sequencing would be too costly.

The inventors have extracted DNA from over 300 of the samples, and the other extractions will be performed using the Qiagen MagAttract^{™} kit on a Qiagen M48 robot. Quantification of DNA will be performed using the Quant-iT dsDNA HS assay kit and Qubit^{™} fluorometer (Invitrogen) prior to plate-based library construction. Libraries of sheared genomic fragments will be constructed in 96 well plates using a Covaris E210 sonication platform and Biomek^{™}FX liquid handler. Library construction begins with 1µg of DNA which is automatically 1) sheared to an average size of ~200bp, 2) transferred to 96 well plates, 3) end-polished, 4) poly-A tailed, 5) ligated to barcoded adapters, and 6) PCR-amplified with oligonucleotides specific for sequences required for clonal cluster generation. Once constructed, libraries will be pooled (up to 94 samples in a single run) and enriched by solid or liquid phase capture probes.

There are competing approaches for target enrichment including using custom Agilent and Nimblegen solid and solution phase capture platforms however, to date, these platforms have not been validated for multiplexed sample capture and we would be required to examine the 400 samples as individual capture experiments, which would be cost prohibitive. Thus, a solid phase microfluidic capture platform developed by febit for the SOLiD^{™} 3.5 sequencing platform (febit biomed gmbh and Applied Biosystems, respectively) will be used. The febit HybSelect^{™} microarray-based capture method selectively captures fragments of sequence from complex genomic libraries through hybridization of DNA samples to specific oligonucleotides generated by light-activated in-situ synthesis on microfluidic chips **(Geniom^{™}** Biochip)⁵¹. Each Geniom^{™} Biochip contains 8 individually addressable arrays, each composed of > 15,000 capture probes segmented into features of variable number and size. The number of features, density, and probe length are customizable, up to a maximum of 800 kb per array. Twelve barcoded SOLiD^{™} sequencing libraries will be pooled for each array (96 libraries per GenioM^{™} Biochip) and subjected to sequence capture, washing and elution on a Geniom^{™} RT device. The sequence capture steps will be performed by febit's Genomics services unit.

The enriched samples will be assessed and quantified using a DNA 1000 series II assay (Agilent) and Quant-iT dsDNA HS assay kit and Qubit^{™} fluorometer, respectively (Invitrogen). Sets of libraries will be further pooled (up to 96 samples per slide) and subjected to bulk emulsion PCR (emPCR), enrichment, and sequencing on the SOLiD^{™} 3.5 platform. Each bulk emPCR will be subjected to a work flow analysis (WFA) run on the SOLiD^{™} platform to ensure that noise to signal ratio are within specification. Once approved, the emPCR will be used for large scale bead deposition targeting ~ 500 million reads per slide, 1 billion reads per run.

**Data Analysis:** Image processing to colour calls will be performed on instrument and resulting files will be aligned to the reference human genome (NCBI build 36.1, hg18) using Bioscope^{™} v1.01 (Applied Biosystems). Variants in the resulting alignments will be detected using the diBayes package (Applied Biosystems). The probability of the existence of a heterozygote or a non-reference homozygote will be evaluated using prior probabilities of the SNP being a "miscolourcall", "position error" or "probe error". In addition, data will be analysed independently of the diBayes approach by aligning all reads in colourspace using the Mosaik aligner (http://bioinformatics.bc.edu/marthlab/Mosaik). This algorithm has several advantages over competing methods: it uses a banded Smith-Waterman approach for alignment that is more likely to detect insertions and deletions, it takes full advantage of the colourspace reads, and may be less prone to misalignment. Moreover, Mosaik seamlessly converts back to base-space and thus allows us to leverage the cancer-specific framework the inventors have developed for SNV detection called SNVMix 56 used in the discovery of the *FOXL2* mutation in granulosa cell tumours of the ovary¹ and the analysis of genome-wide mutational evolution in a lobular breast cancer.²⁵ After alignment, we will predict SNVs and cross reference all non-synonymous protein coding predictions against a database of known SNPs to enrich the results for somatic variants.²⁵ All remaining non-synonymous SNVs and protein coding insertions and deletions will henceforth be referred to as somatic mutation candidates (SMCs). The SMCs will be validated by targeted ultra-deep amplicon sequencing in tumour and normal DNA on Illumina GA_{IIx} machines²⁵. This approach is expected to yield allelic frequency information and is sensitive enough to confirm SMCs, even those present in a small minority of cells. Reads will be aligned to the human reference genome using Maq 0.7.1 and variants will be assessed using a Binomial exact test followed by correction for multiple comparisons using the Benjamini-Hochberg method. All positions where the variant is statistically significantly present in the tumour but not the normal will be considered a validated somatic mutation.

Once the sequencing has been completed, the data will be used to identify and quantify all mutations. Validation of potential mutations will be performed by Illumina sequencing of PCR amplicons from tumour derived DNA.²⁵ Matched normal DNA will be assessed for the presence of all validated mutations to determine somatic versus germline status. It is estimated that there will be five potential mutations per case in the genes sequenced (thus 2000 mutations in 400 cases). The inventors have working primer sets for the known cancer genes and estimate the need to develop an additional 200 primer sets to validate mutations in SWI/SNF genes. Amplicons for all mutations will be placed into two pools, each of which will be used to create a library that will be run on a single lane of the Illumina G_{IIx} analyzer. The amplicons from normal and tumour DNA will be pooled into separate libraries to eliminate the need for barcoding. If identical changes are seen in multiple cases, these will be validated by Sanger sequencing. In cases where *ARID1A* mutations are found, LOH at the second allele will be assessed using FISH.

If the HybSelect method does not work as outlined above, alternative sequencing strategies will be used if needed: either Illumina-based sequencing of selected amplicons or Sanger sequencing will be used. If Sanger based sequencing is used, the number of cases analysed will be decreased to 100 due to increased costs associated with this approach.

### Example 8.2 - Validation of Frequency of BAF250a Expression in Ovarian Cancer

As described above, the inventors have demonstrated that the mutation status of *ARID1A* correlates with BAF250a expression. The above experiments were conducted using a mouse monoclonal antibody directed against a 111 amino acid region (amino acids 1216-1326) C-terminal to the ARID domain of BAF250a (clone 3H2Abgent Inc.). As this antibody targets the central region of the protein, there may be positive staining even when nonsense mutations within the C-terminus give rise to a truncated form of the protein. As several of the mutations identified by the inventors fall within the C-terminus (Figure 2), the inventors are developing a C-terminal specific antibody for BAF250a. The C-terminal specific antibody will be used to re-immunostain all cases. Cases with missense mutations or inframe deletions would not be expected to show loss of BAF250a expression (with either antibody).

### Example 8.3 - Evaluation of Expression Levels of BAF250b

Expression of BAF250b (encoded by *ARID1B*) will also be assessed. Since SWI/SNF complexes cannot contain both BAF250a and BAF250b, it is predicted that depletion of BAF250a may correlate with increased BAF250b.

Based on the RNA-seq data described above, it appears that *ARID1B* expression levels are not affected by mutations in *ARID1A* and in fact are not variable when compared across all cancer types. However, in order to ensure that BAF250b protein expression is not increased due to BAF250a deficiencies, all BAF250a-negative cases will be immunostained for expression of BAF250b. Since SWI/SNF complexes cannot contain both BAF250a and BAF250b, it may be that the absence of BAF250a corresponds to an enrichment of BAF250b containing complexes. This would have functional consequences as BAF250a depletion has been shown to specifically inhibit cell cycle arrest, while BAF250b depletion has no effect on cell cycle arrest⁵². In addition, BRM, BRG1, and BAF47 immunohistochemistry will be done on all tissue microarrays.

Cases with unexplained loss of BAF250a, BRM, BRG1, or BAF47 expression will be re-examined for promoter hypermethylation, which has been described for BRM and BRG126, using primers designed through access to known tools such as http://www.urogene.org/methprimer/index.html or published primers. Immunostaining of all cases will be preformed at the Genetic Pathology Evaluation Centre^{8,14,50}.

### Example 8.4 - Statistical Analysis

With about 150 CCC cases, a determination of the rate of *ARID1A* mutations can be assessed to within ± 10%. Analysis of 400 ovarian cancer tumours will allow detection of differences in mutation rates between pathological or molecularly defined subtypes of 15% (80% power level). Mutation frequency in SWI/SNF genes will be compared between cancer subtypes using Fisher's exact test. It will be determined whether CCCs with *ARID1A* mutations or loss of expression have a distinct clinical phenotype by correlation with patient outcomes and tumour stage. Log rank test and Kaplan Meier plots will be used to assess differences in survival characteristics^{4,50}. Associations with clinical and biomarker data will be assessed with chi-square tests and contingency tables.

### Example 8.5 - Confirm that Mutations in ARID1A are Early Events in Oncogenesis

In all cases where mutations within SWI/SNF genes are found, putative precursor lesions (when present) will be analyzed by immunohistochemistry for BAF250a expression; FISH for chromosomal based LOH; and laser capture microdissection (LCM) followed by Sanger sequencing of cloned PCR products to assess ARID1A mutation status. This approach has already been used on case CCC23 discussed above.

### Example 8.6 - Determination of Functional Consequences of ARID1A Mutations in CCC Derived Cell Models

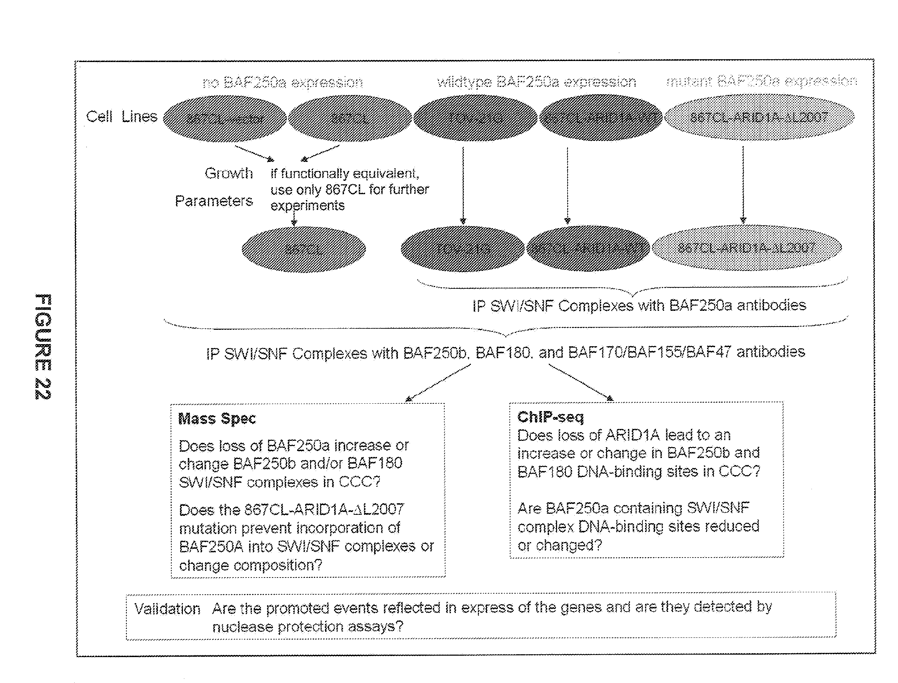

A determination will be made as to how *ARID1A* (wildtype, loss, and mutant) affects cell growth and survival in clear cell carcinoma cells and xenograft mouse models. The effect of *ARID1A* mutations on protein-protein interactions will be determined using co-immunoprecipitation experiments followed by mass spectrometry. To determine if *ARID1A* mutations affect recruitment to BAF250a targets, chromatin immumoprecipitation combined with next generation sequencing will be used (ChIP-seq). Genome-wide nuclease accessibility assays will be used to validate SWI-SNF-chromatin interactions identified in chromatin immunoprecipitation experiments (Figure 22).

The inventors have developed a transplantable xenograft from VOA867 (CCC14), a CCC with a heterozygous *ARID1A* truncating somatic mutation (C 1680A (Y560*)) in exon 3 resulting in complete loss of BAF250a expression. An *ARID1A*-null cell line (867CL) established from the VOA867 (CCC14) xenograft to create isogenic derivatives will be used for all functional studies. Site-directed mutagenesis of the full length *ARID1A* cDNA (pCMV6-XL4 plasmid, OriGene Technologies) will be conducted to generate *ARID1A* constructs corresponding to mutations identified through RNA-seq. Specifically, 876CL isogenic lines will be created with 1) vector only as a control (867CL-vector), 2) the 6018-6020delGCT (2007ΔL) 3 bp deletion found in VOA120 (867CL-ARID1A-ΔL2007), and 3) wildtype ARID1A (867CL-ARID1A-WT). To prevent disruption of BRG1 binding resulting from a BAF250a C-terminal GFP fusion, a vector with GFP expressed through an IRES site (internal ribosome entry site) and use BAF250a antibodies to validate expression. These *ARID1A* mutant and wild-type constructs will be packaged into pLVX-Puro lentiviral expression vector which will be used to infect 867CL cells. Transduced cells will be selected using puromycin and/or flow sorting for GFP. Stable clones will be derived by limited dilution to select clones with *ARID1A* expression that is comparable TOV-21G (a CCC derived cell line that endogenously expresses wildtype *ARID1A*). These cells (867CL, 867CL-vector, 867CL-ARID1A-ΔL2007, 867CL-ARID1A-WT) will be subjected to RNA-seq and differentially expressed genes will be mapped to pathways using Ingenuity Pathway Analysis software. These data will also be used to validate ChIP-seq results.

### Example 8.7 - Effect of ARID1A on Cell Cycle and Growth

The three isogenic and parent 867CL cells will be analyzed in vitro for growth and cell cycle activity. MTT (3-(4,5-dmethylthiazol-2-yl)-2,5-diphenyl tetrazolium bromide) assays will be used to evaluate proliferation status as a function of mitochondrial activity.⁵³ As a second measurement of cell survival, the cell colony formation assay will be used, which assesses cell cycle arrest or cell death leading to reduced colony formation.⁵⁴ As depletion of *ARID1A* plays a role in cell cycle repression,⁵² cell cycle activity will be assessed through analysis of DNA synthesis as measured by [³H] thymidine incorporation into DNA.⁵⁵ To further elucidate the biological function of *ARID1A in vivo*, the parent 867CL cells will be transplaned along with the three derived isogenic cells into NOD/SCID mice using the xenograft sub-renal capsule technique³⁶ and growth properties of the tumour xenografts will be compared. If 867CL-ARID1A-WT xenografts have a longer tumour doubling time compared to ARID1A-null (867CL, 867CL-vector) or ARID1A-mutant (867CL-ARID1A-ΔL2007), this would further support that *ARID1A* acts as a tumour suppressor in CCCs.

If isogenic cell lines cannot successfully be created from 867CL cells, other *ARID1A*-null cells will be selected to serve as potential alternatives: 1) any of the nine CCC cell lines sequenced in the Examples above with loss of *ARID1A* expression or 2) IOSE (immortalized ovarian surface epithelium) or HCT116 cells stably expressing lentiviral *ARID1A* shRNA. Preliminary data demonstrate efficient *ARID1A* shRNA-mediated knock-down of BAF250a expression in HCT116 cells (Figure 8) and cells lacking BAF250a expression will be selected using puromycin and/or flow sorting for GFP.

### Example 8.8 - Immunoprecipitation of SWI/SNF Complexes

The inventors predict that 867CL and 867-vector cells will produce identical results in the cell cycle and growth assays described above. If this is the case, it will be concluded that the vector has no effect and will use only 867CL cells for the remaining experiments. Immunoprecipitation (IP) of SWI/SNF complexes is required for both assessment of protein composition (in MS experiments) and chromatin binding (in ChIP-seq experiments). IP experiments will be done from nuclear extracts in null (867CL), mutant (867CL-ARID1A-ΔL2007) and wildtype *ARID1A* (867CL-ARID1A-WT) cell lines using three SWI/SNF antibodies targeting: 1) one core component of the complex (i.e. BAF155, BAF170, or BAF47)⁴⁹; 2) BAF250b; and 3) BAF180. In addition, the inventors will IP SWI/SNF complexes using BAF250a antibodies from 867CL-ARID1A-ΔL2007, 867CL-ARID1A-WT, and TOV-21G cells (Figure 22).

With reference to Figure 22, initially, five cell lines will be assessed for the effect of *ARID1A* on cell growth: 867CL (no BAF250a expression), 867CL-vector (no BAF250a expression), TOV-21G (clear cell carcinoma derived with endogenous normal ARID1A expression), 867CL-ARID1A-WT (wildtype ARID1A expression in 867CL cells), and 867CL-ARID1A-ΔL2007 (mutant *ARID1A* and BAF250a expression). Assuming the introduction of the empty vector into 867CL cells has no effect, 867CL-vector cells will not be studied further for MS and ChIP-seq experiments. The remaining four cell lines will have SWI/SNF complexes isolated from nuclear extracts through IP of (1) BAF250b, (2) BAF180, and (3) BAF170, BAF155, or BAF47. In addition, those cells with BAF250a expression (either wildtype or mutant) will have SWI/SNF complexes isolated through IP of BAF250a. Protein composition and abundance of various SWI/SNF complexes will be investigated using MS. SWI/SNF binding to chromatin will be investigated using ChIP-seq.

Antibodies for IP⁵⁶ are available from Santa-Cruz (BAF170,sc-10757; BAF47, sc-16189; BAF250a, sc-32761) and Bethy Laboratories (BAF180, A301-590A; BAF155, A301-019A; BAF250b, A301-047A) and these will be tested to select antibodies that produce the cleanest results. As SWI/SNF complexes must contain one of BAF250a, BAF250b, or BAF180, *ARID1A* loss or mutations may manifest as dramatically reduced levels of wildtype BAF250a complexes and an increase in BAF250b or BAF180 containing complexes. A second consequence of *ARID1A* mutations may be alteration of the protein combinations within SWI/SNF complexes. A third consequence of these mutations may be changes in chromatin targets for SWI/SNF complexes which would affect gene regulation. These will all be investigated using the combination of MS and ChIP-seq experiments described below.

### Example 8.9 - The Effects of ARID1A Mutations on SWI/SNF Complex Composition

The inventors will use the multiple reaction monitoring (MRM) MS analysis technique to quantitate signature peptides for 15 known components of SWI/SNF complexes (Figure 1)²⁶ in the IPed SWI/SNF complexes described above. MRM is a quantitative, highly sensitive, triple quadrupole MS scan technique used to quantify MS/MS fragments (termed transitions) emanating from a specific peptide (from a protein of interest).⁵⁶ For the 15 proteins to be measured, an MRM assay will be designed using MS/MS spectra for tryptic peptides obtained from MS spectra databases (http://www.peptideatlas.org/, http://gpmdb.thegpm.org/). One peptide for all SWI/SNF proteins will be selected. Each will be unique in the human proteome and have robust MS/MS signals, except in the case of BAF250a where three peptides (C-terminal, central, and N-terminal) will be selected so that any truncated versions of BAF250a will be detected. MS data will be collected on an ABI 4000QTrap MS which can measure all 17 peptides using 3 transitions per peptide in a single multiplex assay; transitions for each peptide will co-chromatograph in the MS analysis. MultiQuant (ABI) will be used to calculate the signal volume of each transition in the chromatograms. The transitions for each peptide will be summed and used to calculate the relative changes of the SWI/SNF proteins between samples. Values will be normalized for starting cell number, and perform three independent replicate experiments will be performed to allow for statistical analysis.

Comparison of 867CL to 867CL-ARID1A-WT or TOV-21G cells will identify changes associated with altered overall SWI/SNF complex composition and altered BAF250b and BAF180 complex composition associated with ARID1A loss in CCCs. It is predicted that the SWI/SNF composition of 867CL- ARID1A-ΔL2007 compared to 867CL-ARID1A-WT and TOV-21G will identify proteins gained or lost due to BAF250a interactions that are dependent on contacts to Leu2007 or tertiary structures affected by the Leu2007 residue. This will be verified by IP of SWI/SNF complexes using the BAF250a antibody in 867CL-ARID1A-ΔL2007, 867CL-ARID1A-WT, and TOV-21G cells.

IP of SWI/SNF complexes from nuclear extracts using antibodies to SWI/SNF core proteins and analysis by MS/MS has succeeded in identifying all of the core proteins to be monitored^{57,58}, thus the more sensitive MRM technique should also be successful. Technical replicates for MRM analysis vary by less than 5 %, thus it is anticipated that small (10-20%) changes in the relative levels of individual SWI/SNF proteins in the overall pool of SWI/SNF components will be detectable. The experiments will not be able to differentiate between SWI/SNF complexes with different compositions, but should detect major adjustments in SWI/SNF complex composition due to the loss of BAF250a. If the data identify compelling changes, experiments to characterize individual SWI/SNF complexes in the BAF250a mutant lines would be performed. Using biochemical size fractionation chromatography and the MRM assay, the molar stoichiometry of individual SWI/SNF complexes and their components would be determined.

### Example 8.10 - ARID1A Interaction with Chromatin

Experiments will be conducted to determine if mutations in *ARID1A* lead to distinctive SWI/SNF-chromatin interactions. The effect of *ARID1A* mutations on BAF250a mediated transactivation will be assayed using a luciferase reporter construct. ChIP-seq and nuclease protection assays⁵⁹ will assess how wildtype and mutant BAF250a proteins differentially interact with chromatin.

**Effect of *ARIDIA* mutations on transactivation:** The XG46TL plasmid will be obtained that contains multiple glucocorticoid receptor response elements upstream of a luciferase reporter which will be transiently transfected into the four cell lines (867CL, 867CL-ARID1A-WT, 867CL-ARID1A-ΔL2007, TOV-21G). Cells will be treated with dexamethasone to stimulate the glucorticoid receptor which acts in concert with the SWI/SNF complex to activate transcription; this can be assessed through quantitation of luciferase as previously described.⁶⁰ Using this reporter system, effects of *ARID1A* mutations on transactivation can be directly assessed.

**Effect of *ARIDIA* Mutations on BAF250a Interaction with DNA:** The impact of *ARID1A* mutations on SWI/SNF complex binding to chromatin will be assessed using ChIP-seq to identify promoters interacting with SWI/SNF complexes in the four cell lines described above. IP's will be done as described above, in duplicate. The tools required for ChIP-seq and associated analysis have been previously described.⁶¹ The coverage chosen (~ 5 Gbp per library) will achieve the redundancy necessary to find high confidence peaks while maintaining budget constraints.

Cell lines will be treated with formaldehyde to cross-link DNA and associated proteins. Cleared cell lysates will be sonicated to shear the chromatin, then incubated with the selected SWI/SNF antibody followed by overnight Protein A/G Sepharose precipitation. Chromatin IPs will be washed, eluted, used to create an Illumina sequencing library, and sequenced in one lane of an Illumina flow cell. Paired reads will be aligned to the reference human genome with Exonerate (http://www.ebi.ac.uk/ ~ guy/exonerate) or Maq.⁶² Regions of clustered sequence tags (peaks) corresponding to chromatin will be defined using FindPeaks software.⁶³ Sequences not present in both biological replicates or found to be in common with the ARID1A-wildtype (867CL-ARID1A-WT, TOV21G), ARID1A-mutant (867CL-ARID1A-ΔL2007, and ARID1A-null (867CL) cells will be removed from analysis. Data will be analysed with MEME⁶⁴ to detect any over-represented motifs and with TRANSFAC to find known transcription factor binding sites. Finally, genes and highly conserved intergenic sites will be identified proximal to peaks. It is expected to see on the order of 1000 peaks at false discovery rate = 0.05. These areas will be prioritized based on where they are located (i.e. promoter regions upstream of target genes), the relevance of genes that may be transcriptionally regulated by these regions, and by the data obtained from the targeted sequencing and MS experiments.

This approach will allow identification of high confidence DNA-protein interactions in the primary dataset and eliminate signals due to sporadic or non-specific DNA-protein binding. Interactions of interest will be validated with orthogonal techniques including interactions of BAF250a with selected promoters upstream of a luciferase reporter gene. To determine whether findings from the ChIP-seq experiments are supported by expression changes for the implicated genes, data generated from triplicate libraries from the 867CL, 867CL-vector, 867CL-ARID1A-WT, and 867CL-ARID1A-ΔL2007cell lines which will be analysed by RNA-seq for differential gene expression using the edgeR Bioconductor statistical package.⁶⁵ Briefly, edgeR models read count data for a particular gene according to a negative Binomial distribution. Using an overdispersed Poisson model for differential gene expression analysis, the model is able to account for both technical and biological variation. All genes showing differential expression and concomitant differential ChIP-seq peak detection in their promoter regions will be selected as candidate genes affected by *ARID1A* mutation.

**Effects of *ARID1A* mutations on *in vivo* nuclesome remodelling:** Nucleosome-free DNA is sensitive to digestion by low concentrations of nuclease and *ARID1A* mutations may be reflected as changes in nuclease sensitivity. Nuclease sensitivity at 20 *ARID1A* targets identified through ChIP-seq will be assessed, focusing on genes that are known drug targets or cancer genes and for which the ChIP-seq data correspond to changes in gene expression through RNA-seq. Briefly, nuclei from CCC cell lines will be treated with low concentrations of micrococcal nuclease or DNAaseI, causing only DNA from nucleosome-free regions to be degraded. The remaining protected DNA will be sequenced using primers specific for each target.

In the event that no changes in SWI/SNF composition or DNA binding are identified in the presence of *ARID1A* mutations, it will additionally be assessed whether these mutations result in alteration of histone ubiquitination, as it was recently demonstrated that BAF250b (the gene product of *ARID1B*) is an E3 ubquitin ligase for histone H2B at lysine (K)120.⁶⁶

### Example 8.11 - Identification of Therapeutic Targets in CCC with ARID1A Mutations

An siRNA library will be used to identify genes that are necessary for survival of cells expressing mutant *ARID1A.* Any identified genes would be potential targets for the development of therapeutics for clear cell cancers with *ARIDIA* mutations. The siRNA library will be screened in xenograft mouse models of *ARID1A* mutant clear cell carcinomas.

An established approach to identifying therapeutic targets in cancer, is to search for "synthetic lethality", also known as conditional genetics. The prototype example of synthetic lethality is PARP inhibition in the context of BRCA1 or BRCA2 deficiency^{67,68}. To define therapeutic targets that would be uniquely effective in tumours bearing *ARID1A* mutations, a synthetic lethal (viability) screen will be conducted using established siRNA/high content screening methodology. A fully integrated siRNA screening facility equipped with robotics, fluid handling and an INCELL 1100 high content imager. The inventors will use a published siRNA/high content multiparameter screening method⁶⁹ to measure seven phenotypic parameters relevant to cell viability, proliferation, cell cycle, and associated checkpoints.

The siRNA libraries screened will be the Hannon/Elledge lenti-shRNA human library (approx 66,000 constructs) and the Dharmacon siGenome pools, representing approximately 22,000 gene loci. Both libraries have been internally formatted for 96 well and 384 well screens. In preference, the siRNA library pools will be used for screening at 25nM. If for any reason siRNA transfection proves difficult, the shRNA library will be used. The 867CL, 867CL-ARID1A-ΔL2007, 867CL-ARID1A-WT cells will be used. If screening using these cell lines proves intractable, an isogenic knockout of *ARID1A* in HCT116 cells will be used as a second choice (Figure 8).

Cell lines will be compared pairwise, in 384 well plates. Each transfection plate will contain controls for transfection efficiency, transfection toxicity and siRNA effectiveness, and phenotypic baseline measurements. In the primary screens, all 22,000 siRNA pools/66000 shRNAs (representing the full human gene complement thus far established) will be used. The screen will be performed in 384 well plates on the three isogenic cell lines, in triplicate. Control plates (all wells transfected with the same non-targeting siRNA) will be used to correct for well position effects in a linear mixed effects model. Cells will be transduced with 25 nM of siRNA pools or lentiviral particles at a MOI of 3, as appropriate. The effects of each siRNA pool or shRNA on cell viability, cell shape and transduction efficiency will be measured 4 days post transfection. Transduction efficiency will be evaluated using control wells from each screen plate, containing PLK1 siRNA. All conditions will be assessed in triplicate to allow adequate assessment of variability. After image segmentation and quantification as described, the data will be analysed with a linear mixed effects model⁷⁰ to handle known screening artefacts such as wellplate edge effects, reagent dispenser pipette tip effects etc. Multiple comparisons adjustments will be performed using the Benjamini-Hochberg approach for p-values, and empirical Bayes shrinkage for effect estimates where appropriate.^{71,72} To measure the degree of synthetic interaction, an interaction index (scaled ratio of wt phenotype size to mutant phenotype size, for a given siRNA) will be calculated from linear model adjusted values. The top 5% of candidate shRNA targets, based on ranked synthetic effect magnitude and ranked p-value, will be triaged for follow-up validation. Following primary screening and selection of initial hits, these will be rescreened individually (pool deconvolution) for maximum discrimination. Re-validated siRNAs will also be assayed in conjunction with qRT-PCR (quantitative reverse trasncriptase PCR) for the target transcript to determine whether the phenotype segregates with the degree of transcript knockdown. siRNAs surviving these filters will be grouped by GO-terms and structural class, for further follow up.

While a number of exemplary aspects and embodiments have been discussed above, those of skill in the art will recognize certain modifications, permutations, additions and sub-combinations thereof. It is therefore intended that the following appended claims and claims hereafter introduced are not limited by the preferred embodiments set forth in the disclosure and the examples, but are to be given the broadest interpretation consistent with the specification as a whole.

### References

1. Shah SP, Kobel M, Senz J, Morin RD, Clarke BA, Wiegand KC, Leung G, Zayed A, Mehl E, Kalloger SE, Sun M, Giuliany R, Yorida E, Jones S, Varhol R, Swenerton KD, Miller D, Clement PB, Crane C, Madore J, Provencher D, Leung P, DeFazio A, Khattra J, Turashvili G, Zhao Y, Zeng T, Glover JN, Vanderhyden B, Zhao C, Parkinson CA, Jimenez-Linan M, Bowtell DD, Mes-Masson AM, Brenton JD, Aparicio SA, Boyd N, Hirst M, Gilks CB, Marra M, Huntsman DG. N Engl J Med Mutation of FOXL2 in granulosa-cell tumors of the ovary 2009;360:2719-29
2. Kobel M, Kalloger SEH, D. G., Santos J, Swenerton KD, Seidman JD, Gilks CB. Int J Gynecol Pathol Differences in tumor cell type in low versis high stage ovarian carcinomas;in press
3. Gilks CB, Prat J. Hum Pathol Ovarian carcinoma pathology and genetics: recent advances 2009;40:1213-23
4. Kobel M, Kalloger SE, Boyd N, McKinney S, Mehl E, Palmer C, Leung S, Bowen NJ, Ionescu DN, Rajput A, Prentice LM, Miller D, Santos J, Swenerton K, Gilks CB, Huntsman D. PLoS Med Ovarian Carcinoma Subtypes Are Different Diseases: Implications for Biomarker Studies 2008;5:e232
5. Crotzer DR, Sun CC, Coleman RL, Wolf JK, Levenback CF, Gershenson DM. Gynecol Oncol Lack of effective systemic therapy for recurrent clear cell carcinoma of the ovary 2007;105:404-8
6. Goff BA, Sainz de la Cuesta R, Muntz HG, Fleischhacker D, Ek M, Rice LW, Nikrui N, Tamimi HK, Cain JM, Greer BE, Fuller AF, Jr. Gynecol Oncol Clear cell carcinoma of the ovary: a distinct histologic type with poor prognosis and resistance to platinum-based chemotherapy in stage III disease 1996;60:412-7
7. Sugiyama T, Kamura T, Kigawa J, Terakawa N, Kikuchi Y, Kita T, Suzuki M, Sato I, Taguchi K. Cancer Clinical characteristics of clear cell carcinoma of the ovary: a distinct histologic type with poor prognosis and resistance to platinum-based chemotherapy 2000;88:2584-9
8. Press JZ, De Luca A, Boyd N, Young S, Troussard A, Ridge Y, Kaurah P, Kalloger SE, Blood KA, Smith M, Spellman PT, Wang Y, Miller DM, Horsman D, Faham M, Gilks CB, Gray J, Huntsman DG. BMC Cancer Ovarian carcinomas with genetic and epigenetic BRCA1 loss have distinct molecular abnormalities 2008;8:17
9. Gilks CB. J Oncol Molecular abnormalities in ovarian cancer subtypes other than high-grade serous carcinoma 2010;epub ahead of print
10. Gilks CB, Ionescu DN, Kalloger SE, Kobel M, Irving J, Clarke B, Santos J, Le N, Moravan V, Swenerton K. Hum Pathol Tumor cell type can be reproducibly diagnosed and is of independent prognostic significance in patients with maximally debulked ovarian carcinoma 2008;39:1239-51
11. Leitao MM, Jr., Boyd J, Hummer A, Olvera N, Arroyo CD, Venkatraman E, Baergen RN, Dizon DS, Barakat RR, Soslow RA. Am J Surg Pathol Clinicopathologic analysis of early-stage sporadic ovarian carcinoma 2004;28:147-59
12. Pectasides D, Pectasides E, Psyrri A, Economopoulos T. Oncologist Treatment issues in clear cell carcinoma of the ovary: a different entity? 2006;11:1089-94
13. Tavassoli FA, Devilee P. World Health Organization of Tumours: Pathology and genetics of tumours of the breast and female genital organs. Lyon: IARCpress; 2003.
14. Kobel M, Kalloger SE, Carrick J, Huntsman D, Asad H, Oliva E, Ewanowich CA, Soslow RA, Gilks CB. Am J Surg Pathol A limited panel of immunomarkers can reliably distinguish between clear cell and high-grade serous carcinoma of the ovary 2009;33:14-21
15. Kuo KT, Mao TL, Jones S, Veras E, Ayhan A, Wang TL, Glas R, Slamon D, Velculescu VE, Kuman RJ, Shih Ie M. Am J Pathol Frequent activating mutations of PIK3CA in ovarian clear cell carcinoma 2009;174:1597-601
16. Campbell IG, Russell SE, Choong DY, Montgomery KG, Ciavarella ML, Hooi CS, Cristiano BE, Pearson RB, Phillips WA. Cancer Res Mutation of the PIK3CA gene in ovarian and breast cancer 2004;64:7678-81
17. Kolasa IK, Rembiszewska A, Felisiak A, Ziolkowska-Seta I, Murawska M, Moes J, Timorek A, Dansonka-Mieszkowska A, Kupryjanczyk J. Cancer Biol Ther PIK3CA amplification associates with resistance to chemotherapy in ovarian cancer patients 2009;8:21-6
18. Wang Y, Helland A, Holm R, Kristensen GB, Borresen-Dale AL. Hum Mutat PIK3CA mutations in advanced ovarian carcinomas 2005;25:322
19. Willner J, Wurz K, Allison KH, Galic V, Garcia RL, Goff BA, Swisher EM. Hum Pathol Alternate molecular genetic pathways in ovarian carcinomas of common histological types 2007;38:607-13
20. Kurman RJ, Shih Ie M. Int J Gynecol Pathol Pathogenesis of ovarian cancer: lessons from morphology and molecular biology and their clinical implications 2008;27:151-60
21. Scully RE, Young RH, Clement PB. Tumors of the ovary, maldeveloped gonads, fallopian tube, and broad ligament 1998:141
22. Yamamoto S, Tsuda H, Suzuki K, Takano M, Tamai S, Matsubara O. Virchows Arch An allelotype analysis indicating the presence of two distinct ovarian clear-cell carcinogenic pathways: endometriosis-associated pathway vs. clear-cell adenofibroma-associated pathway 2009;455:261-70
23. Yamamoto S, Tsuda H, Takano M, Hase K, Tamai S, Matsubara O. J Pathol Clear-cell adenofibroma can be a clonal precursor for clear-cell adenocarcinoma of the ovary: a possible alternative ovarian clear-cell carcinogenic pathway 2008;216:103-10
24. Aparicio SA, Huntsman DG. J Pathol Does massively parallel DNA resequencing signify the end of histopathology as we know it? ;220:307-15
25. Shah SP, Morin RD, Khattra J, Prentice L, Pugh T, Burleigh A, Delaney A, Gelmon K, Guliany R, Senz J, Steidl C, Holt RA, Jones S, Sun M, Leung G, Moore R, Severson T, Taylor GA, Teschendorff AE, Tse K, Turashvili G, Varhol R, Warren RL, Watson P, Zhao Y, Caldas C, Huntsman D, Hirst M, Marra MA, Aparicio S. Nature Mutational evolution in a lobular breast tumour profiled at single nucleotide resolution 2009;461:809-13
26. Reisman D, Glaros S, Thompson EA. Oncogene The SWI/SNF complex and cancer 2009;28:1653-68
27. Sif S, Saurin AJ, Imbalzano AN, Kingston RE. Genes Dev Purification and characterization of mSin3A-containing Brg1 and hBrm chromatin remodeling complexes 2001;15:603-18
28. Wang W, Xue Y, Zhou S, Kuo A, Cairns BR, Crabtree GR. Genes Dev Diversity and specialization of mammalian SWI/SNF complexes 1996;10:2117-30
29. Wang X, Nagl NG, Wilsker D, Van Scoy M, Pacchione S, Yaciuk P, Dallas PB, Moran E. Biochem J Two related ARID family proteins are alternative subunits of human SWI/SNF complexes 2004;383:319-25
30. Inoue H, Furukawa T, Giannakopoulos S, Zhou S, King DS, Tanese N. J Biol Chem Largest subunits of the human SWI/SNF chromatin-remodeling complex promote transcriptional activation by steroid hormone receptors 2002;277:41674-85
31. Trotter KW, Fan HY, Ivey ML, Kingston RE, Archer TK. Mol Cell Biol The HSA domain of BRG1 mediates critical interactions required for glucocorticoid receptor-dependent transcriptional activation in vivo 2008;28:1413-26
32. Nie Z, Xue Y, Yang D, Zhou S, Deroo BJ, Archer TK, Wang W. Mol Cell Biol A specificity and targeting subunit of a human SWI/SNF family-related chromatin-remodeling complex 2000;20:8879-88
33. Maher CA, Kumar-Sinha C, Cao X, Kalyana-Sundaram S, Han B, Jing X, Sam L, Barrette T, Palanisamy N, Chinnaiyan AM. Nature Transcriptome sequencing to detect gene fusions in cancer 2009;458:97-101
34. Maher CA, Palanisamy N, Brenner JC, Cao X, Kalyana-Sundaram S, Luo S, Khrebtukova I, Barrette TR, Grasso C, Yu J, Lonigro RJ, Schroth G, Kumar-Sinha C, Chinnaiyan AM. Proc Natl Acad Sci U S A Chimeric transcript discovery by paired-end transcriptome sequencing 2009;106:12353-8
35. Goya R, Sun MG, Morin RD, Leung G, Ha G, Wiegand KC, Senz J, Crisan A, Marra MA, Hirst M, Huntsman D, Murphy KP, Aparicio S, Shah SP. Bioinformatics SNVMix: predicting single nucleotide variants from next generation sequencing of tumors 2010; 26:730-6
36. Press JZ, Kenyon JA, Xue H, Miller MA, De Luca A, Miller DM, Huntsman DG, Gilks CB, McAlpine JN, Wang YZ. Gynecol Oncol Xenografts of primary human gynecological tumors grown under the renal capsule of NOD/SCID mice show genetic stability during serial transplantation and respond to cytotoxic chemotherapy 2008;110:256-64
37. Bengtsson H, Ray A, Spellman P, Speed TP. A single-sample method for normalizing and combining full-resolution copy numbers from multiple platforms, labs and analysis methods. Bioinformatics 2009;25:861-7
38. Conrad DF, Pinto D, Redon R, et al. Origins and functional impact of copy number variation in the human genome. Nature;464:704-12
39. Rozen S, Skaletsky H. Primer3 on the WWW for general users and for biologist programmers. Methods Mol Biol 2000; 132:365-86
40. Hochberg Y, Benjamini Y. More powerful procedures for multiple significance testing. Stat Med 1990;9:811-8
41. Dagan T, Talmor Y, Graur D. Ratios of radical to conservative amino acid replacement are affected by mutational and compositional factors and may not be indicative of positive Darwinian selection. Mol Biol Evol 2002;19:1022-5
42. Makretsov N, He M, Hayes M, et al. A fluorescence in situ hybridization study of ETV6-NTRK3 fusion gene in secretory breast carcinoma. Genes Chromosomes Cancer 2004;40:152-7
43. Chang YF, Imam JS, Wilkinson MF. The nonsense-mediated decay RNA surveillance pathway. Annu REv Biochem 2007;76:51-74
44. Ness RB. Endometriosis and ovarian cancer: thoughts on shared pathophysiology. Am J Obstet Gynecol 2003;189:280-94
45. Viganó P, Somigliana E, Chiodo I, Abbiati A, Vercellini P. Molecular mechanisms and biological plausibility underling the malignant transformation of endometriosis: a critical analysis. Hum Reprod Update 2006;12:77-89
46. Alkushi A, Clarke BA, Akbari M, et al. Identification of prognostically relevant and reproducible subsets of endometrial adenocarcinoma based on clustering analysis of immunostaining data. Mod Pathol 2007; 20: 1156-1165
47. Wiegand KC, Shah SP, Al-Agha OM, et al. ARID1A mutations in endometriosis-associated ovarian carcinomas. N Engl J Med 2010; 363: 1532-1543
48. Gao X, Tate P, Hu P, Tjian R, Skarnes WC, Wang Z. Proc Natl Acad Sci U S A ES cell pluripotency and germ-layer formation require the SWI/SNF chromatin remodeling component BAF250a 2008;105:6656-61
49. Weissman B, Knudsen KE. Cancer Res Hijacking the chromatin remodeling machinery: impact of SWI/SNF perturbations in cancer 2009;69:8223-30
50. Kobel M, Xu H, Bourne PA, Spaulding BO, Shih Ie M, Mao TL, Soslow RA, Ewanowich CA, Kalloger SE, Mehl E, Lee CH, Huntsman D, Gilks CB. Mod Pathol IGF2BP3 (IMP3) expression is a marker of unfavorable prognosis in ovarian carcinoma of clear cell subtype 2009;22:469-75
51. Bau S, Schracke N, Kranzle M, Wu H, Stahler PF, Hoheisel JD, Beier M, Summerer D. Anal Bioanal Chem Targeted next-generation sequencing by specific capture of multiple genomic loci using low-volume microfluidic DNA arrays 2009;393:171-5
52. Nagl NG, Jr., Patsialou A, Haines DS, Dallas PB, Beck GR, Jr., Moran E. Cancer Res The p270 (ARID1A/SMARCF1) subunit of mammalian SWI/SNF-related complexes is essential for normal cell cycle arrest 2005;65:9236-44
53. Choi JH, Choi KC, Auersperg N, Leung PC. Endocr Relat Cancer Differential regulation of two forms of gonadotropin-releasing hormone messenger ribonucleic acid by gonadotropins in human immortalized ovarian surface epithelium and ovarian cancer cells 2006;13:641-51
54. Franken NA, Rodermond HM, Stap J, Haveman J, van Bree C. Nat Protoc Clonogenic assay of cells in vitro 2006;1:2315-9
55. Nagl NG, Jr., Wang X, Patsialou A, Van Scoy M, Moran E. Embo J Distinct mammalian SWI/SNF chromatin remodeling complexes with opposing roles in cell-cycle control 2007;26:752-63
56. Ryme J, Asp P, Bohm S, Cavellan E, Farrants AK. J Cell Biochem Variations in the composition of mammalian SWI/SNF chromatin remodelling complexes 2009;108:565-76
57. Ho L, Ronan JL, Wu J, Staahl BT, Chen L, Kuo A, Lessard J, Nesvizhskii AI, Ranish J, Crabtree GR. Proc Natl Acad Sci U S A An embryonic stem cell chromatin remodeling complex, esBAF, is essential for embryonic stem cell self-renewal and pluripotency 2009;106:5181-6
58. Lessard J, Wu JI, Ranish JA, Wan M, Winslow MM, Staahl BT, Wu H, Aebersold R, Graef IA, Crabtree GR. Neuron An essential switch in subunit composition of a chromatin remodeling complex during neural development 2007;55:201-15
59. Woo CJ, Kharchenko PV, Daheron L, Park PJ, Kingston RE. Cell A region of the human HOXD cluster that confers polycomb-group responsiveness; 140:99-110
60. Tse R, Marroquin BA, Dorscheid DR, White SR. Am J Physiol Lung Cell Mol Physiol Beta-adrenergic agonists inhibit corticosteroid-induced apoptosis of airway epithelial cells 2003;285:L393-404
61. Robertson G, Hirst M, Bainbridge M, Bilenky M, Zhao Y, Zeng T, Euskirchen G, Bernier B, Varhol R, Delaney A, Thiessen N, Griffith OL, He A, Marra M, Snyder M, Jones S. Nat Methods Genome-wide profiles of STAT1 DNA association using chromatin immunoprecipitation and massively parallel sequencing 2007;4:651-7
62. Li H, Ruan J, Durbin R. Genome Res Mapping short DNA sequencing reads and calling variants using mapping quality scores 2008;18:1851-8
63. Fejes AP, Robertson G, Bilenky M, Varhol R, Bainbridge M, Jones SJ. Bioinformatics FindPeaks 3.1: a tool for identifying areas of enrichment from massively parallel short-read sequencing technology 2008;24:1729-30
64. Bailey TL, Boden M, Buske FA, Frith M, Grant CE, Clementi L, Ren J, Li WW, Noble WS. Nucleic Acids Res MEME SUITE: tools for motif discovery and searching 2009;37:W202-8
65. Robinson MD, McCarthy DJ, Smyth GK. Bioinformatics edgeR: a Bioconductor package for differential expression analysis of digital gene expression data;26:139-40
66. Li XS, Trojer P, Matsumura T, Treisman JE, Tanese N. Mol Cell Biol Mammalian SWI/SNF-A subunit BAF250/ARID1 is an E3 ubiquitin ligase that targets histone H2B;epub ahead of print
67. Farmer H, McCabe N, Lord CJ, Tutt AN, Johnson DA, Richardson TB, Santarosa M, Dillon KJ, Hickson I, Knights C, Martin NM, Jackson SP, Smith GC, Ashworth A. Nature Targeting the DNA repair defect in BRCA mutant cells as a therapeutic strategy 2005;434:917-21
68. Bryant HE, Schultz N, Thomas HD, Parker KM, Flower D, Lopez E, Kyle S, Meuth M, Curtin NJ, Helleday T. Nature Specific killing of BRCA2-deficient tumours with inhibitors of poly(ADP-ribose) polymerase 2005;434:913-7
69. Poon SS, Wong JT, Saunders DN, Ma QC, McKinney S, Fee J, Aparicio SA. Cytometry A Intensity calibration and automated cell cycle gating for high-throughput image-based siRNA screens of mammalian cells 2008;73:904-17
70. Ghosh D, Chinnaiyan AM. Funct Integr Genomics Covariate adjustment in the analysis of microarray data from clinical studies 2005;5:18-27
71. Benjamini Y, Drai D, Elmer G, Kafkafi N, Golani I. Behav Brain Res Controlling the false discovery rate in behavior genetics research 2001;125:279-84
72. Ghosh D, Chinnaiyan AM. Biom J Empirical Bayes identification [correction of identication] of tumor progression genes from microarray data 2007;49:68-77

## Claims

1. A method for determining whether endometriosis of a subject is likely to progress to clear cell ovarian carcinoma, endometrioid carcinoma, endometrial carcinoma, or uterine carcinoma; for determining the prognosis for a subject suffering from clear cell ovarian carcinoma, endometrioid carcinoma, endometrial carcinoma, or uterine carcinoma; and/or for determining whether standard chemotherapeutic agents are likely to be effective in treating clear cell ovarian carcinoma, endometrioid carcinoma, endometrial carcinoma, or uterine carcinoma; the method comprising assaying a sample of the endometriosis or carcinoma for expression of BAF250a, wherein the absence of expression of BAF250a indicates a likelihood that the endometriosis will progress to clear cell ovarian carcinoma, endometrioid carcinoma, endometrial carcinoma, or uterine carcinoma, indicates a poor prognosis, or indicates that the standard chemotherapeutic agents are not likely to be effective.

2. A method as defined in claim 1, wherein the step of assaying the sample for expression of BAF250a comprises immunohistochemistry using an antibody specific for BAF250a.

3. A method for determining whether endometriosis of a subject is likely to progress to clear cell ovarian carcinoma, endometrioid carcinoma, endometrial carcinoma, or uterine carcinoma; for determining a prognosis for a subject suffering from clear cell ovarian carcinoma, endometrioid carcinoma, endometrial carcinoma, or uterine carcinoma; and/or for determining whether standard chemotherapeutic agents are likely to be effective in treating clear cell ovarian carcinoma, endometrioid carcinoma, endometrial carcinoma, or uterine carcinoma; the method comprising assaying a sample of the endometriosis or carcinoma for the presence of mutations in the *ARID1A* gene in the sample, wherein the presence of a significant mutation in the *ARID1A* gene indicates a likelihood that the endometriosis will progress to clear cell ovarian carcinoma, endometrioid carcinoma, endometrial carcinoma, or uterine carcinoma, indicates a poor prognosis, or indicates that the standard chemotherapeutic agents are not likely to be effective, wherein the significant mutation comprises a mutation in the DNA sequence of the *ARID1A* gene that produces a mutated BAF250a protein that is not able to fully perform the typical function of BAF250a.

4. A method as defined in claim 3, wherein the step of assaying for the presence of mutations in the *ARID1A* gene comprises sequencing the *ARID1A* gene or the mRNA produced from the *ARID1A* gene.

5. A method as defined in any one of claims 3 or 4, wherein the step of assaying for the presence of mutations in the *ARID1A* gene comprises using a mutation detection method, and wherein the mutation detection method optionally comprises using a PCR-based detection method or fluorescence in-situ hybridization.

6. A method as defined in any one of claims 3, 4 or 5, wherein the mutation in the *ARID1A* gene comprises a nonsense mutation or a significant missense mutation, wherein a significant missense mutation comprises a mutation in the DNA sequence of the *ARID1A* gene that produces a mutated BAF250a protein that is not able to fully perform the typical function BAF250a.

7. A method as defined in any one of claims 3, 4, 5 or 6, wherein the mutation in the *ARID1A* gene comprises one of the mutations set forth in SEQ ID NO.:2 through SEQ ID NO.:122.

8. A method as defined in any one of claims 1 to 7, wherein the standard chemotherapeutic agents comprise platinum or taxane therapies.

9. A method as defined in any one of claims 1 to 8, wherein the ovarian carcinoma is clear cell carcinoma of the ovary.

## Patentansprüche

1. Verfahren zur Bestimmung, ob Endometriose eines Patienten wahrscheinlich zu klarzelligem Ovarialkarzinom, endometrioidem Karzinom, Endometriumkarzinom oder Gebärmutterkarzinom fortschreitet; zur Bestimmung der Prognose für einen Patienten, der an klarzelligem Ovarialkarzinom, endometrioidem Karzinom, Endometriumkarzinom oder Gebärmutterkarzinom leidet; und/oder zur Bestimmung, ob standardmäßige Chemotherapeutika für die Behandlung von klarzelligem Ovarialkarzinom, endometrioidem Karzinom, Endometriumkarzinom oder Gebärmutterkarzinom wahrscheinlich wirksam sind; wobei das Verfahren die Untersuchung einer Probe der Endometriose oder des Karzinoms auf die Expression von BAF250a umfasst, wobei die Abwesenheit der Expression von BAF250a Folgendes anzeigt: eine Wahrscheinlichkeit, dass die Endometriose zu klarzelligem Ovarialkarzinom, endometrioidem Karzinom, Endometriumkarzinom oder Gebärmutterkarzinom fortschreiten wird; eine schlechte Prognose oder, dass die standardmäßigen Chemotherapeutika wahrschlich nicht wirksam sind.

2. Verfahren nach Anspruch 1, wobei der Schritt der Untersuchung der Probe für die Expression von BAF250a Immunhistochemie unter Verwendung eines Antikörpers umfasst, der für BAF250a spezifisch ist.

3. Verfahren zur Bestimmung, ob Endometriose eines Patienten wahrscheinlich zu klarzelligem Ovarialkarzinom, endometrioidem Karzinom, Endometriumkarzinom oder Gebärmutterkarzinom fortschreitet; zur Bestimmung einer Prognose für einen Patienten, der an klarzelligem Ovarialkarzinom, endometrioidem Karzinom, Endometriumkarzinom oder Gebärmutterkarzinom leidet; und/oder zur Bestimmung, ob standardmäßige Chemotherapeutika für die Behandlung von klarzelligem Ovarialkarzinom, endometrioidem Karzinom, Endometriumkarzinom oder Gebärmutterkarzinom wahrscheinlich wirksam sind; wobei das Verfahren das Untersuchen einer Probe der Endometriose oder des Karzinoms auf die Anwesenheit von Mutationen im Gen ARID1A in der Probe umfasst, wobei die Anwesenheit einer signifikanten Mutation in dem Gen ARID1A Folgendes anzeigt: eine Wahrscheinlichkeit, dass die Endometriose zu klarzelligem Ovarialkarzinom, endometrioidem Karzinom, Endometriumkarzinom oder Gebärmutterkarzinom fortschreiten wird; eine schlechte Prognose oder, dass die standardmäßigen Chemotherapeutika wahrschlich nicht wirksam sind, wobei die signifikante Mutation eine Mutation in der DNA-Sequenz des Gens ARID1A umfasst, die ein mutiertes Protein BAF250a erzeugt, das nicht in der Lage ist, die typische Funktion von BAF250a vollständig zu erfüllen.

4. Verfahren nach Anspruch 3, wobei der Schritt der Untersuchung auf die Anwesenheit von Mutationen in dem Gen ARID1A die Sequenzierung des Gens ARID1A oder der mRNA, die von dem Gen ARID1A erzeugt wird, umfasst.

5. Verfahren nach einem der Ansprüche 3 oder 4, wobei der Schritt des Untersuchens auf die Anwesenheit von Mutationen in dem Gen ARID1A die Verwendung eines Mutationsnachweisverfahrens umfasst und wobei das Mutationsnachweisverfahren optional die Verwendung eines PCRbasierten Nachweisverfahrens oder einer In-Situ-Fluoreszenzhybridisierung umfasst.

6. Verfahren nach einem der Ansprüche 3, 4 oder 5, wobei die Mutation in dem Gen ARID1A eine Nonsense-Mutation oder eine signifikante Missense-Mutation umfasst, wobei eine signifikante Missense-Mutation eine Mutation in der DNA-Sequenz des Gens ARID1A umfasst, die ein mutiertes Protein BAF250a erzeugt, das nicht in der Lage ist, die typische Funktion von BAF250a vollständig auszuführen.

7. Verfahren nach einem der Ansprüche 3, 4, 5 oder 6, wobei die Mutation in dem Gen ARID1A eine der Mutationen umfasst, die in SEQ ID NO:. 2 bis SEQ ID NO:. 122 aufgeführt sind.

8. Verfahren nach einem der Ansprüche 1 bis 7, wobei die standardmäßigen Chemotherapeutika Platin- oder Taxan-Therapien umfassen.

9. Verfahren nach einem der Ansprüche 1 bis 8, wobei das Ovarialkarzinom ein klarzelliges Karzinom des Eierstocks ist.

## Revendications

1. Procédé de détermination que l'endométriose d'une patiente est susceptible ou non de progresser au stade de carcinome ovarien à cellules claires, de carcinome endométrioïde, de carcinome endométrial, ou de carcinome utérin ; de détermination du pronostic pour une patiente souffrant de carcinome ovarien à cellules claires, de carcinome endométrioïde, de carcinome endométrial, ou de carcinome utérin ; et/ou de détermination que des agents chimiothérapeutiques standard sont susceptibles ou non d'être efficaces dans le traitement de carcinome ovarien à cellules claires, carcinome endométrioïde, carcinome endométrial, ou carcinome utérin ; le procédé comprenant l'analyse d'un échantillon de l'endométriose ou du carcinome à la recherche d'une expression de BAF250a, dans lequel l'absence d'expression de BAF250a indique une probabilité que l'endométriose va progresser au stade de carcinome ovarien à cellules claires, de carcinome endométrioïde, de carcinome endométrial, ou de carcinome utérin, indique un pronostic défavorable, ou indique que les agents chimiothérapeutiques standard sont susceptibles de ne pas être efficaces.

2. Procédé selon la revendication 1, dans lequel l'étape d'analyse de l'échantillon à la recherche d'une expression de BAF250a comprend l'immunohistochimie utilisant un anti-corps spécifique à la BAF250a.

3. Procédé de détermination que l'endométriose d'une patiente est susceptible ou non de progresser au stade de carcinome ovarien à cellules claires, de carcinome endométrioïde, de carcinome endométrial, ou de carcinome utérin ; de détermination d'un pronostic pour une patiente souffrant de carcinome ovarien à cellules claires, de carcinome endométrioïde, de carcinome endométrial, ou de carcinome utérin ; et/ou de détermination que des agents chimiothérapeutiques standard sont susceptibles ou non d'être efficaces dans le traitement de carcinome ovarien à cellules claires, carcinome endométrioïde, carcinome endométrial, ou carcinome utérin ; le procédé comprenant l'analyse d'un échantillon de l'endométriose ou du carcinome à la recherche de la présence de mutations du gène ARID1A dans l'échantillon, dans lequel la présence d'une mutation importante du gène ARID1A indique une probabilité que l'endométriose va progresser au stade de carcinome ovarien à cellules claires, de carcinome endométrioïde, de carcinome endométrial, ou de carcinome utérin, indique un pronostic défavorable, ou indique que les agents chimiothérapeutiques standard sont susceptibles de ne pas être efficaces, dans lequel la mutation importante comprend une mutation dans la séquence d'ADN du gène ARID1A qui produit une protéine BAF250a mutée qui ne peut pas remplir pleinement la fonction caractéristique de la protéine BAF250a.

4. Procédé selon la revendication 3, dans lequel l'étape d'analyse à la recherche de la présence de mutations dans le gène ARID1A comprend le séquençage du gène ARID1A ou de l'ARNm produit à partir du gène ARID1A.

5. Procédé selon l'une quelconque des revendications 3 ou 4, dans lequel l'étape d'analyse à la recherche de la présence de mutations dans le gène ARID1A comprend l'utilisation d'un procédé de détection de mutation, et dans lequel le procédé de détection de mutation comprend optionnellement l'utilisation d'un procédé reposant sur la PCR ou une hybridation in-situ en fluorescence.

6. Procédé selon l'une quelconque des revendications 3, 4 ou 5, dans lequel la mutation dans le gène ARID1A comprend une mutation non-sens ou une mutation faux sens importante, dans lequel une mutation faux sens importante comprend une mutation de la séquence d'ADN du gène ARID1A qui produit une protéine BAF250a mutée qui ne peut pas remplir pleinement la fonction caractéristique de la protéine BAF250a.

7. Procédé selon l'une quelconque des revendications 3, 4, 5 ou 6, dans lequel la mutation dans le gène ARID1A comprend l'une des mutations représentées dans SEQ ID N°2 à SEQ ID N°122.

8. Procédé selon l'une quelconque des revendications 1 à 7, dans lequel les agents chimiothérapeutiques standard comprennent des thérapies à base de platine ou de taxanes.

9. Procédé selon l'une quelconque des revendications 1 à 8, dans lequel le carcinome ovarien est un carcinome ovarien à cellules claires.
